(19) Europäisches Patentamt
European Patent Office
Office européen des brevets

(11) EP 3 253 323 B1

(12) **FASCICULE DE BREVET EUROPEEN**

(45) Date de publication et mention de la délivrance du brevet:
**17.07.2024 Bulletin 2024/29**

(21) Numéro de dépôt: **16705060.8**

(22) Date de dépôt: **05.02.2016**

(51) Classification Internationale des Brevets (IPC):
***A61B 34/30*** *(2016.01)* ***B25J 9/16*** *(2006.01)*
***B25J 18/00*** *(2006.01)* ***A61B 34/37*** *(2016.01)*
***A61B 34/00*** *(2016.01)*

(52) Classification Coopérative des Brevets (CPC):
**A61B 34/37; A61B 34/30; A61B 34/75; A61B 34/76; A61B 34/77; B25J 18/007;**
A61B 2090/062; A61B 2090/067

(86) Numéro de dépôt international:
**PCT/EP2016/052534**

(87) Numéro de publication internationale:
**WO 2016/124752 (11.08.2016 Gazette 2016/32)**

(54) **DISPOSITIF D'ASSISTANCE À LA MANIPULATION D'UN INSTRUMENT**

VORRICHTUNG ZUR UNTERSTÜTZUNG DES BETRIEBS EINES INSTRUMENTS

DEVICE TO ASSIST WITH THE OPERATION OF AN INSTRUMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.02.2015 FR 1550921**

(43) Date de publication de la demande:
**13.12.2017 Bulletin 2017/50**

(73) Titulaires:
- **Sorbonne Université**
  **75006 Paris (FR)**
- **Centre National de la Recherche Scientifique (CNRS)**
  **75016 Paris (FR)**
- **Endocontrol**
  **38700 La Tronche (FR)**

(72) Inventeurs:
- **MOREL, Guillaume**
  **75013 Paris (FR)**
- **DONG, Lin**
  **Yantai**
  **Shandong 265110 (CN)**
- **RICHER, Florian**
  **94370 Sucy En Brie (FR)**
- **PERRIN, Nicolas**
  **75013 Paris (FR)**
- **VIDAL, Clément**
  **38000 Grenoble (FR)**
- **BARDOU, Bérengère**
  **38000 Grenoble (FR)**

(74) Mandataire: **Regimbeau**
**20, rue de Chazelles**
**75847 Paris Cedex 17 (FR)**

(56) Documents cités:

| | |
|---|---|
| **WO-A2-02/060653** | **WO-A2-02/060653** |
| **WO-A2-2006/124390** | **WO-A2-2006/124390** |
| **WO-A2-2006/124390** | **US-A1- 2007 142 823** |
| **US-A1- 2007 142 823** | **US-B1- 6 786 896** |
| **US-B1- 6 786 896** | **US-B1- 6 786 896** |

- **ANONYMOUS: "Lever - Wikipedia", 31 January 2016 (2016-01-31), XP093086281, Retrieved from the Internet <URL:https://en.wikipedia.org/w/index.php?title=Lever&oldid=702525681> [retrieved on 20230927]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 253 323 B1

## Description

### DOMAINE TECHNIQUE GENERAL

L'invention concerne la robotique de co-manipulation, c'est-à-dire la manipulation simultanée d'un instrument par un robot et par un opérateur afin d'assister l'opérateur manipulant l'instrument. Ici le mot instrument désigne un objet en général, qui peut être un outil, une pièce mécanique, un appareil de visualisation tel qu'une caméra, etc. L'utilisateur effectue une tâche qui consiste à manipuler cet objet et le robot de co-manipulation a pour rôle de l'aider.
L'invention concerne plus précisément les procédés et dispositifs d'assistance à la manipulation d'un instrument passant à travers un orifice dont il faut contrôler l'extrémité distale. De tels dispositifs ou procédés sont avantageusement utilisés en coopération avec un contrôle vidéo issu d'un endoscope afin que l'opérateur puisse observer en direct ses manipulations.
L'invention trouve notamment application en chirurgie laparoscopique pour laquelle des instruments longs et fins sont insérés (*via* des canules) dans le corps d'un patient afin de permettre une intervention chirurgicale.
Ce type d'opérations nécessite généralement plusieurs instruments et peut durer plusieurs heures.

### ETAT DE L'ART

Dans le présent texte, les exemples seront donnés pour le domaine chirurgical. Néanmoins, ils peuvent s'appliquer sans difficulté à tout type de manipulation à travers un orifice et nécessitant des équipements similaires à ceux d'une laparoscopie (mécanique de précision, etc.).
En l'absence de dispositif d'assistance à la manipulation d'un instrument tel qu'un robot portant un instrument, tous les instruments sont manipulés par le chirurgien et/ou un assistant. La chirurgie laparoscopique impose généralement des gestes précis et techniques, ainsi qu'une posture corporelle difficile et soutenue pour l'opérateur, qui peut engendrer des troubles musculo-squelettiques.
Une évolution de cette chirurgie s'est dirigée vers une robotisation télémanipulée, où le robot qui porte les instruments est manipulé à distance par un chirurgien. Outre le coût élevé de l'installation d'un tel robot, il existe des limitations importantes. On peut citer par exemple le fait que le chirurgien manipulateur n'est pas « stérile » (il travaille sur une console non stérile), ou encore que les opérations péri-chirurgicales sont compliquées (le chirurgien doit indiquer exactement quel instrument il souhaite et à quel endroit, puisqu'il n'est pas directement sur le corps du patient), ou encore le fait qu'un tel robot ne fonctionne que pour des opérations très localisées (prostate, utérus, etc.) mais ne permet pas d'opérer efficacement lorsqu'il faut atteindre des zones du corps éloignées entre elles, par exemple lorsqu'il faut changer de canule (dispositif permettant l'insertion de l'instrument dans le patient).
Une autre évolution, qui est celle qui intéresse l'invention, s'est dirigée vers une co-manipulation d'un outil par l'intermédiaire d'un robot et d'un opérateur. Le robot, dans ce cas, outre le fait qu'il porte les instruments, permet de filtrer les imprécisions du geste de l'opérateur (vibrations, etc.).
Des procédés de filtrage utilisent par exemple une viscosité, en établissant un rapport entre une vitesse de déplacement et une force, ou bien une raideur, en établissant un rapport entre un écart de position et une force, ou bien en attribuant une inertie virtuelle à l'instrument. Des combinaisons de ces effets peuvent aussi être recherchées. L'ensemble de ces effets et de leurs combinaisons possibles font partie des impédances mécaniques réalisables par le robot grâce à des moyens de programmation.
Comme représenté en **figure 1,** les dispositifs d'assistance 1 pour co-manipulation d'un instrument présentent trois degrés de liberté actifs, ce qui peut se faire par exemple grâce à trois actionneurs successifs A1, A2, A3 qui contrôlent chacun un axe, ce qui permet à un point donné de la dernière extrémité du robot contrôlée par ces actionneurs de se déplacer dans les trois dimensions de l'espace. Il est possible, sans changer le principe de l'invention, d'organiser les trois actionneurs en parallèle et non en série, selon les principes de la robotique parallèle connus de l'homme de l'art. Il est également possible d'utiliser plus que trois actionneurs pour déplacer un point donné de la dernière extrémité du robot, constituant ainsi une chaîne cinématique redondante, selon les principes des robots redondants connus de l'homme de l'art. En outre, ces dispositifs présentent trois degrés de liberté passifs, grâce à une rotule passive disposée sur ladite extrémité. Cette dernière ne peut donc pas transmettre de moment et permet la manipulation libre de l'instrument 20 par un opérateur. L'instrument 20 est par ailleurs inséré de façon à ce que l'axe de l'instrument 20 passe par le centre de la rotule passive. On définit un point d'attache P qui passe par le centre de la liaison rotule et qui passe par l'instrument 20.
Le dispositif est contrôlé par une unité de traitement U. L'architecture générale consiste à récupérer des informations (position, vitesse généralement) depuis l'instrument 20, à les traiter et à déterminer une force à appliquer au point d'attache.
Des capteurs C1, C2, C3 sont agencés au niveau de chaque actionneur (donc trois capteurs), et deux capteurs C4, C5 sont agencés au niveau de la rotule pour mesurer les rotations autres que celle qui se fait autour de l'axe de

l'instrument. Autrement dit, C4 et C5 permettent de mesurer l'orientation de l'axe de l'instrument. Pour réaliser le dispositif, il n'est pas nécessaire de connaître la rotation de l'instrument selon son axe propre.

En effet, les points d'intérêt que sont le centre de la rotule, la position de la main de l'opérateur ou la pointe de l'instrument (« *tip* », ou extrémité distale de l'instrument) sont tous situés sur l'axe de l'instrument. La **figure 3** illustre le positionnement de ces points.

Mais les dispositifs de co-manipulation connaissent actuellement des limitations qui nuisent à leur utilisation ou qui empêchent une meilleure exploitation des possibilités qu'ils pourraient conférer.

Certaines de ces limitations sont les suivantes.

Comme mentionné précédemment, des impédances sont implémentées pour améliorer les sensations de l'opérateur lors de la manipulation et aussi pour perfectionner les gestes.

Une solution connue est de créer une impédance au niveau du centre de la rotule, ce qui est facilité par le fait que la position du centre de la rotule est précisément connue à chaque instant grâce aux trois capteurs C1, C2 et C3 présents au niveau des trois actionneurs.

Mais cette solution n'est pas optimale du point de vue des sensations de l'opérateur, et il peut être préférable de créer une impédance programmable au niveau de la poignée de saisie de l'instrument (dans la main de l'opérateur) ou au niveau de l'extrémité distale de l'instrument, qui est la partie que l'opérateur souhaite contrôler.

Or pour programmer une impédance sur les autres points, il est nécessaire, selon l'état de l'art, d'exploiter les capteurs C4 et C5 de la liaison rotule en plus des capteurs C1, C2 et C3 des articulations.

Pour des raisons techniques, les capteurs des actionneurs offrent un signal précis avec peu de bruit alors que les capteurs de la rotule (typiquement des potentiomètres) peuvent être de moindre qualité et offrent un signal peu précis avec beaucoup de bruit.

Par conséquent, la connaissance des données relatives à d'autres points de l'axe de l'instrument (l'extrémité distale de l'instrument ou un point de la poignée proximale par exemple) telles que la position ou la vitesse est relativement mauvaise, ce qui ne permet pas d'avoir l'impédance voulue à l'endroit voulu.

Un autre problème qui se pose dans la robotique d'assistance à la chirurgie laparoscopique (ou plus généralement à la manipulation au travers d'un orifice) réside dans le paramétrage du robot et en particulier le paramétrage du point d'incision, qui est le point d'entrée de l'instrument dans le corps du patient.

Certains robots existants pour l'assistance à la chirurgie laparoscopique fonctionnent autour d'un point fixe. Il faut alors précisément positionner le robot, avant l'opération, de façon à faire correspondre ledit point fixe avec le point d'incision.

Une procédure de recalage, effectuée préalablement au commencement de l'opération est nécessaire.

De telles procédures sont sujettes à des erreurs et ralentissent le déroulement de l'intervention. Il est donc souhaitable de proposer des dispositifs qui ne nécessite ni positionnement précis du robot avant l'intervention ni recalage.

Un autre problème se trouve dans l'application des impédances et le ressenti de l'opérateur. Dans le cas de la viscosité, qui établit un rapport entre une force et une vitesse (pour donner l'impression au chirurgien de se déplacer dans un milieu visqueux), il est judicieux que l'opérateur, pour des mouvements légers et précis, travaille avec une grande viscosité pour filtrer toutes ses imprécisions malgré des vitesses petites ; en revanche, lorsqu'il doit déplacer l'instrument sur une autre zone du patient, il doit travailler avec une petite viscosité, sinon il va devoir exercer une force importante pour simplement déplacer l'outil, ce qui peut provoquer une fatigue inutile et une perte de temps, le robot ralentissant le geste.

Pour ce faire, il a déjà été proposé de générer une viscosité variable entre deux états (faible quand la vitesse de la main est grande, grande quand la vitesse de la main est faible), ou continue.

De telles configurations peuvent générer des instabilités, comme il est reporté sur un essai expérimental illustré sur de la **figure 4** : l'opérateur souhaite ici déplacer l'instrument avec une vitesse moyenne, qui correspond à une viscosité intermédiaire entre la valeur maximale (pour des vitesses lentes) et la valeur minimale (pour des vitesses rapides). Pour atteindre cette vitesse moyenne en partant d'une vitesse nulle, l'opérateur va forcer pour accélérer le déplacement. Lorsque la vitesse augmente, la chute de la viscosité va créer une diminution de la résistance du robot ce qui va avoir pour effet d'accélérer brutalement le déplacement ; par conséquent la vitesse devient rapidement plus grande que la vitesse moyenne à laquelle l'opérateur souhaite déplacer l'instrument. L'opérateur diminue alors sa force pour ralentir, ce qui va provoquer l'augmentation de la viscosité et décélérer brutalement le déplacement : il est donc créé une oscillation autour de la vitesse moyenne désirée par l'utilisateur, qui empêche une utilisation correcte du système de co-manipulation.

Pour offrir à l'opérateur un robot de co-manipulation qui l'aide d'une façon plus dirigée, il est possible de définir des guides. Pour ce faire, il faut d'abord définir des contraintes géométriques (un point, une droite, une sphère, etc.). Le robot de co-manipulation est alors programmé pour appliquer des efforts destinés à ramener un point de l'instrument sur cette contrainte, ou au contraire à repousser un point de l'instrument lorsqu'il s'approche de cette contrainte. Une façon connue de l'état de l'art est d'utiliser des forces élastiques (c'est-à-dire proportionnelles au déplacement par rapport à la contrainte) ce qui est une forme particulière d'impédance mécanique. Ceci est particulièrement intéressant,

dans le cadre de l'application visée, si le point de l'instrument est son extrémité distale car cela permet de guider cette extrémité, par exemple pour éviter une région anatomique données, ou pour maintenir l'extrémité dans un plan de résection. Dans ce cas, il est possible de définir la contrainte relativement à l'anatomie du patient, puis de calculer cette contrainte dans le système de coordonnées du robot grâce à un procédé connu de recalage. Mais cette opération est complexe car elle demande une planification et un recalage.

Enfin, les opérations durant plusieurs heures, il arrive que l'opérateur ne soit pas à proprement parler en train de manipuler l'instrument. Par exemple, il peut souhaiter reposer son bras, ou effectuer un autre geste avec un autre instrument. Il arrive aussi que la manipulation consiste à simplement maintenir constantes la position et l'orientation de l'instrument. C'est le cas par exemple lorsque l'instrument est un écarteur avec lequel l'opérateur soulève (ou écarte) un organe afin de laisser libre l'accès à une partie de l'anatomie que sinon il recouvre. Dans ces divers cas de figure, il est souhaitable que le robot de co-manipulation puisse passer dans un mode dit de verrouillage, qui consiste simplement à immobiliser l'instrument sans assistance de l'opérateur. Commuter dans un mode de verrouillage, ou plus généralement commuter d'un mode de fonctionnement (avec une impédance donnée) à un autre (avec une autre impédance), se fait généralement en utilisant un moyen de commande annexe telle qu'un bouton, une pédale, une commande vocale ou tout autre moyen. L'intégration de ladite commande est problématique dans de nombreuses situations : les mains du chirurgien sont occupées par les instruments ce qui rend complexe l'utilisation de commandes manuelles ; ses pieds sont souvent occupés par des pédales de divers appareils, comme ceux des systèmes d'imagerie ou des instruments électro-chirurgicaux ; sa voix lui sert à communiquer avec le reste de l'équipe et la charge cognitive d'une communication verbale avec une machine, en utilisant un langage spécifique, peut-être perturbante. Il est donc souhaitable que le robot de co-manipulation soit capable de « deviner » dans quel mode l'opérateur souhaite qu'il commute pour réaliser cette commutation.

L'art antérieur pertinent est divulgué dans les documents WO 2006/124390 A2, US 6 786 896 B1, US 2007/142823 A1, WO 02/060653 A2 et "Lever - Wikipedia", 31 janvier 2016 (2016-01-31),Extrait de l'Internet:URL:https://en.wikipedia.org/w/index.php? title=Lever&oldid=702525681.

PRESENTATION DE L'INVENTION

L'invention est limitée par la portée de la revendication indépendante 1. D'autres modes de réalisation sont divulgués dans les revendications dépendantes.

Un but de l'invention est de proposer un procédé non-revendiqué d'assistance permettant d'augmenter la précision du geste de l'opérateur lorsqu'il manipule le dispositif et de proposer justement ledit dispositif.

Selon un premier aspect, ce but est atteint grâce à un procédé d'assistance à la manipulation d'un instrument au moyen d'un dispositif d'assistance à la manipulation de l'instrument,

le dispositif comprenant

un bras articulé destiné à être fixé à un bâti et manipulable par un opérateur, sur lequel un instrument peut être fixé au niveau d'un point d'attache dudit bras articulé formant une liaison rotule passive entre le bras articulé et l'instrument, le bras articulé comprenant des motorisations pour déplacer le point d'attache dans un référentiel lié au bâti, l'instrument étant manipulable autour d'un point d'appui ayant une position connue et fixe dans le référentiel,

une unité de traitement comprenant un processeur configurée pour piloter les motorisations afin de produire une impédance donnée à un point quelconque d'un axe d'instrument reliant le point d'attache au point d'appui ;

le procédé étant caractérisé en qu'il comprend les étapes consistant à :

déterminer des données relatives à une position et/ou une vitesse du point d'attache dans le référentiel lié au dispositif d'assistance;

déterminer un effort à appliquer au point d'attache en fonction desdites données relatives au point d'attache, de la position du point d'appui, et de la distance connue du point d'attache au point quelconque, et d'une impédance donnée à conférer au point quelconque,

piloter des motorisations pour transmettre ledit effort à l'instrument au niveau du point d'attache.

Grâce à un tel procédé, il n'est pas nécessaire d'obtenir des données fournies par les capteurs angulaires au niveau de la liaison rotule passive et il est possible de n'utiliser que des données issues des capteurs des actionneurs dont la mesure est précise et sans bruit (voir introduction). Pour cela une solution consiste à exploiter un modèle de levier autour du point d'appui de l'instrument dont la position est connue. Ce point d'appui est typiquement situé au niveau de la canule (insérée au préalable par exemple dans la paroi abdominale du patient), dans laquelle est glissé

l'instrument.

En connaissant les données relatives au point d'attache et la position du point d'appui, qui est fixe, on peut produire une impédance donnée en un point quelconque de l'axe, comme il sera expliqué dans la description détaillée de l'invention.

Selon un deuxième aspect, l'invention se rapporte à un procédé non-revendiqué de recalage automatique lors de l'assistance à la manipulation d'un instrument au moyen d'un dispositif d'assistance à la manipulation de l'instrument,

le dispositif comprenant

un bras articulé destiné à être fixé à un bâti et manipulable par un opérateur, sur lequel un instrument peut être fixé au niveau d'un point d'attache dudit bras articulé formant une liaison rotule passive entre le bras articulé et l'instrument, le bras articulé comprenant des articulations et des capteurs mesurant le déplacement, ledit bras permettant de déplacer le point d'attache dans un référentiel lié au bâti, l'instrument ayant un axe d'instrument dont la direction est connue,
une unité de traitement comprenant un processeur configurée pour piloter les motorisations ;

le procédé étant caractérisé par les étapes de :

obtention, dans le référentiel lié au dispositif, d'une pluralité de droites définies par l'axe d'instrument, les droites correspondant à une pluralité de configurations de l'instrument,
estimation de l'existence d'une zone d'intersection de ladite pluralité de droites,
obtention de la position centrale de ladite zone si elle existe, ladite zone correspondant alors à un point d'appui de l'instrument.

Un tel procédé est auto-paramétré puisqu'il n'est pas nécessaire de lancer une routine de paramétrage préalablement à l'utilisation de l'instrument. En effet, tant que l'opérateur n'a pas inséré l'instrument dans une canule (qui forme un point d'appui de l'instrument), aucune zone d'intersection n'est détectée et il est considéré qu'il n'y a pas de point d'appui. Une des conséquences peut être le maintien en mode libre, sans impédance particulière appliquée, ou alors avec une viscosité faible. En revanche, dès que l'opérateur insère l'instrument dans une canule, le processeur détecte automatiquement une zone d'intersection et identifie un point d'appui avec ses coordonnées.

Le manque de précision de la position du point d'appui dû aux capteurs (en particulier les capteurs de la rotule passive) est surmonté par la multitude de mesures prises. Ainsi, à l'aide d'une moyenne ou d'un filtrage, il est possible d'obtenir quasiment en continu une valeur précise, avec peu de bruit, pour cette position.

La fréquence des mesures est de l'ordre de la milliseconde, ce qui signifie qu'en moins d'une seconde, plusieurs centaines d'équations ont été obtenues. De cette façon, la co-manipulation est exploitable quasi-instantanément, même lorsque l'opérateur vient d'insérer l'instrument dans la canule.

De plus, le procédé peut fonctionner de façon continue afin d'estimer en permanence la zone d'intersection des droites. Ainsi, le système pourra détecter rapidement lorsque l'opérateur retire l'instrument d'une canule, ou le place dans une autre canule, ou encore lorsque le centre de la canule est déplacé pendant l'opération, par exemple lorsque le patient est repositionné par l'opérateur.

L'intérêt du procédé est alors principalement caractérisé en ceci qu'il ne demande pas une procédure spéciale de recalage mais se réalise en continu, sans intervention particulière de l'opérateur.

Selon un troisième aspect, l'invention se rapporte à un procédé non-revendiqué d'assistance à la manipulation d'un instrument au moyen d'un dispositif d'assistance à la manipulation de l'instrument,

le dispositif comprenant

un bras articulé destiné à être fixé à un bâti et manipulable par un opérateur, sur lequel un instrument peut être fixé au niveau d'un point d'attache dudit bras articulé comprenant des motorisations pour déplacer le point d'attache dans un référentiel lié au bâti,
une unité de traitement comprenant un processeur configurée pour piloter les motorisations ;

le procédé étant caractérisé en qu'il comprend les étapes consistant à :

déterminer la vitesse instantanée d'un point de l'instrument dans le référentiel lié au dispositif d'assistance;
déterminer une première viscosité fonction décroissante de ladite vitesse instantanée,
déterminer une deuxième viscosité à partir de la première viscosité grâce à un procédé de filtrage possédant au moins un paramètre permettant de régler la dynamique du procédé

déterminer un effort au niveau dudit point de l'instrument, fonction :

de ladite vitesse instantanée, et
de la deuxième valeur de viscosité,

piloter des motorisations pour transmettre ledit effort à l'instrument au niveau du point d'attache.

Le filtrage de la viscosité permet de ralentir les variations de force lorsque la vitesse varie et ainsi de supprimer les effets d'instabilité.
La mesure de la vitesse peut avantageusement être elle-même filtrée lorsqu'elle présente des bruits importants.
Le procédé peut avantageusement être appliqué à un dispositif comprenant une rotule passive dont le centre coïncide avec le point d'attache et tel que l'instrument passe par un point fixe. Dans ce cas, le point de calcul de la vitesse et des forces du procédé peut être soit ledit point d'attache soit un autre point de l'axe d'instrument. Dans le cas où le procédé est appliqué à point de l'axe d'instrument qui n'est pas le point d'attache, le premier aspect de l'invention peut se combiner avantageusement avec le troisième aspect de l'invention pour éviter l'usage de capteurs intégrés à la rotule passive dans le calcul des vitesses et des forces.
Selon un quatrième aspect, l'invention se rapporte à un procédé d'assistance à la manipulation d'un instrument au moyen d'un dispositif d'assistance à la manipulation de l'instrument,

le dispositif comprenant

un bras articulé destiné à être fixé à un bâti et manipulable par un opérateur, sur lequel un instrument peut être fixé au niveau d'un point d'attache dudit bras articulé, le bras articulé comprenant des motorisations pour déplacer le point d'attache dans un référentiel lié au dispositif d'assistance, ledit instrument présentant un point d'intérêt,
une unité de traitement comprenant un processeur configurée pour piloter les motorisations ;

le procédé étant caractérisé en qu'il comprend les étapes consistant à :

faire coïncider le point d'intérêt avec des points de l'espace et déterminer la position desdits points de l'espace dans le référentiel lié au dispositif d'assistance ;
construire une contrainte géométrique définie par lesdits points de l'espace à l'aide desdites positions.

Le point d'intérêt de l'instrument peut avantageusement être son extrémité distale, le procédé revenant alors, pour l'opérateur, à pointer des points de l'espace avec l'extrémité distale de l'instrument. Ces points sont ceux que l'opérateur considère comme le point servant à définir la contrainte géométrique.
La contrainte géométrique peut prendre n'importe quelle forme que l'on peut définir par une équation que doit ensuite vérifier le point d'intérêt. En outre, selon ce quatrième aspect de l'invention, il peut ensuite être prévu une étape de détermination d'un effort au niveau du point d'intérêt de l'instrument à l'aide d'une détermination de la distance entre le point d'intérêt de l'instrument, déterminé à un instant quelconque durant la manipulation, et la contrainte géométrique (plan, sphère, point, etc.) par projection orthogonale, l'effort au niveau du point d'intérêt étant fonction d'un coefficient de raideur et ladite distance.
De cette façon, le pilotage des motorisations contraint l'instrument à se positionner par rapport au plan en provoquant l'attraction ou la répulsion dudit point d'intérêt par rapport audit plan. Il peut être nécessaire pour cela de calculer un effort au point d'attache en fonction de l'effort au niveau du point d'intérêt, par exemple lorsque le dispositif possède une rotule passive et l'instrument passe par un point fixe. Les calculs se font alors de façon similaire à ce qui est décrit dans le premier aspect de l'invention, en choisissant pour point quelconque le point d'intérêt de l'instrument.
Selon un cinquième aspect, l'invention se rapporte à un procédé non-revendiqué d'assistance à la manipulation d'un instrument au moyen d'un dispositif d'assistance à la manipulation de l'instrument,

le dispositif comprenant

un bras articulé destiné à être fixé à un bâti et manipulable par un opérateur, sur lequel un instrument peut être fixé au niveau d'un point d'attache dudit bras articulé, le bras articulé comprenant des motorisations pour déplacer le point d'attache dans un référentiel lié au bâti,
une unité de traitement comprenant un processeur configurée pour piloter les motorisations,
une pluralité de modes de commande implémentées dans l'unité de traitement par le processeur, caractérisés par des impédances prédéterminées;

le procédé étant caractérisé en qu'il comprend les étapes consistant à commuter automatiquement d'un mode de commande à un autre lorsqu'un critère est vérifié.

Avantageusement, ledit critère n'exploitera que des mesures fournies par le bras articulé.
En particulier, deux modes de commande peuvent être implémentés :

- un mode verrouillé pour lequel une impédance de verrouillage prédéterminée est appliquée à l'instrument et garantit son maintien en position de l'instrument,
- un mode libre pour lequel une impédance de liberté prédéterminée est appliquée à l'instrument et permet sa manipulation par l'opérateur.

Dans ce cas, le critère de verrouillage peut être une immobilité de l'instrument pendant une durée déterminée et le critère de déverrouillage peut être une translation de l'instrument selon un axe d'instrument.
Les procédés selon les différents aspects peuvent avantageusement être mis en oeuvre seuls ou en combinaison afin de pallier les limitations de l'art antérieur présentées en introduction.
En outre, l'invention propose un dispositif d'assistance pour la manipulation d'un instrument, ledit dispositif étant configuré pour, à l'aide d'une unité de traitement, mettre en oeuvre l'un des procédés décrits précédemment.
Comme indiqué précédemment, ces procédés et dispositifs trouvent application dans tout domaine de la robotique nécessitant une précision de manipulation importante.

PRESENTATION DES FIGURES

D'autres caractéristiques, buts et avantages de l'invention ressortiront de la description qui suit, qui est purement illustrative et non limitative, et qui doit être lue en regard des dessins annexés, sur lesquels :

- la figure 1 est une représentation schématique d'un dispositif d'assistance conforme à un mode de réalisation de l'invention,
- La figure 2 est une représentation schématique illustrant des flux d'information transitant au sein du dispositif,
- La figure 3 illustre différents points caractéristiques d'un instrument manipulé au moyen du dispositif,
- La figure 4 illustre la vitesse du point d'attache avec différents modèle de viscosité, pour un instrument manipulé avec le dispositif,
- Les figures 5a et 5b illustrent différentes positions de manipulation au moyen du dispositif avec notamment un déplacement tangentiel par bras de levier de l'instrument et un déplacement en translation,
- La figure 6 illustre une pluralité de droites passant par une canule dans laquelle est inséré un instrument manipulé au moyen du dispositif,
- La figure 7 illustre un ensemble de plans définis au moyen du dispositif,
- Les figures 8a, 8b et 8c illustrent des critères de déverrouillage d'un mode verrouillé du dispositif.

Sur l'ensemble des figures, les éléments similaires portent des références identiques.

DESCRIPTION DETAILLEE

Dans la présente demande, les positions sont définies par rapport à un repère lié au bâti auquel est fixé le dispositif, ce qui signifie que le robot peut être déplacé sans qu'aucun reparamétrage ne soit nécessaire.
Comme représenté en **figure 1**, un mode de réalisation du dispositif d'assistance 1 comprend un bras articulé 10 fixé à un bâti B. Au niveau d'un point d'attache P dudit bras, un instrument 20 est fixé.
L'instrument 20 est co-manipulé par un opérateur et par le dispositif 1. Il peut s'agir par exemple d'un instrument de chirurgie laparoscopique tel qu'un porte-aiguille, une pince ou un ciseau.
Le bras articulé 10 est motorisé par trois motorisations M1, M2, M3, au niveau des trois articulations A1, A2, A3, ce qui permet de déplacer le point d'attache P selon trois degrés de liberté en translation dans un référentiel R lié au bâti B.
Le bras articulé peut, grâce à ses motorisations M1, M2, M3 amener le point d'attache P n'importe où dans l'espace à la portée du dispositif 1.
Par conséquent, le bras articulé 10 est adapté pour transmettre une force selon les trois directions de l'espace au niveau du point d'attache P.
Il est possible d'utiliser plus de trois moteurs et trois articulations pour positionner le point P et transmettre une force au point P.
Selon le premier aspect de l'invention, la liaison au niveau du point d'attache P est une liaison de type rotule

passive. Par conséquent, le bras articulé 10 ne peut pas transmettre de moment au point P à l'instrument 20, qui est libre de tourner autour du point P par rapport au bras articulé 10.

Cette liaison peut être réalisée par exemple par trois corps successifs, chacun des corps étant relié au corps qui le précède par une liaison pivot et les axes. Dans ce cas, l'axe de l'instrument X-X' peut avantageusement coïncider avec l'axe du dernier pivot.

Une unité de traitement U, comprenant un processeur U1 et une mémoire de stockage U2, contrôle les motorisations M1, M2, M3 et traite les différentes données relatives à l'utilisation dudit dispositif 1.

L'instrument 20 est sous une forme longiligne et s'étend selon un axe principal dit axe d'instrument X-X'.

Des capteurs C1, C2, C3, au niveau de chacune des trois articulations permettent de connaître le mouvement de chacune articulation et de ce fait, grâce à l'unité de traitement U, de connaître la position du point d'attache P. Cette connaissance peut être quasi-continue.

Il est techniquement réalisable d'utiliser dans ces articulations des capteurs qui offrent une très bonne précision et peu de bruit. En effet, généralement, les articulations motorisées possèdent un étage de transmission, qui augmente le couple tout en diminuant la vitesse. Ainsi, un capteur placé sur l'arbre du moteur possèdera une résolution plus importante que s'il devait être placé directement sur l'arbre de sortie de l'articulation.

Avantageusement, le dispositif 1 comprend au moins deux capteurs de position angulaire C4, C5 au niveau du point d'attache P qui mesurent, en combinaison avec les capteurs C1, C2, C3, l'orientation de l'axe d'instrument X-X' dans le référentiel lié au bâti B. Seuls deux capteurs sont nécessaires puisque la rotation de l'instrument 20 selon son axe d'instrument X-X' ne modifie pas l'orientation de l'axe d'instrument X-X'. Lorsque la rotule passive est réalisée avec trois liaisons pivot successives, l'axe de la dernière liaison pivot coïncidant avec l'axe d'instrument X-X', il est avantageux d'utiliser C4 pour mesurer l'angle de la première liaison pivot et C5 pour mesurer l'angle de la deuxième liaison pivot.

Pour des raisons techniques, ces capteurs angulaires C4, C5 offrent un signal peu précis et avec du bruit. Il est actuellement difficile d'implémenter des capteurs de meilleures qualités à cet endroit car pour augmenter la résolution il faudrait par exemple intégrer un étage de réduction qui augmenterait l'encombrement et le poids du dispositif de rotule passive.

Par exemple, les capteurs angulaires C4, C5 sont généralement des potentiomètres.

En outre, il est possible de prévoir un autre capteur C6 qui mesure la rotation de l'instrument 20 selon son axe X-X'. Ceci permet de calculer, en combinaison avec C1, C2, C3, C4 et C5, la position d'un point quelconque de l'instrument qui ne serait pas sur l'axe d'instrument XX'. Ceci présente un avantage par exemple en chirurgie dans le cas des instruments orientés ou flexibles.

De la même façon, on peut prévoir un moteur M6 appliquant un couple selon l'axe d'instrument X-X', qui permet, en combinaison avec les moteurs M1, M2 et M3, et dans le cas où l'axe d'instrument X-X' passe par un point d'appui fixe F connu, d'appliquer des efforts en un point quelconque de l'instrument qui n'appartient pas à son axe X-X'.

L'instrument 20 est adapté pour pénétrer dans le corps d'un patient 60 au niveau d'une canule 30. La canule 30 sert de point d'appui F autour duquel l'instrument est manipulé.

En conséquence, au niveau de la canule 30, l'instrument 20 est libre de coulisser en translation, et libre de pivoter autour du point d'appui F défini par la canule 30.

Par définition, le point d'appui F est sensiblement fixe dans la base B mais peut bouger le long de l'instrument 20 lors des opérations.

Plusieurs points d'intérêt peuvent être observés sur un tel instrument 20 (voir **figure 3)** :

- Le point d'attache P, présenté précédemment, en lequel le bras articulé peut transmettre des force (pas de moment), et réciproquement,
- Le point relatif à la poignée H (« hand » en anglais), qui correspond à l'endroit où l'opérateur tient l'outil, c'est-à-dire l'extrémité proximale de l'instrument,
- Le point relatif à la pointe T (« tip » en anglais), qui correspond à l'extrémité distale de l'instrument et qui agit sur le patient,
- Le point d'appui F (« fulcrum » en anglais), présenté précédemment.

Pour améliorer le rendu d'utilisation à l'opérateur, le dispositif d'assistance 1 permet d'appliquer des impédances à l'instrument, c'est-à-dire qu'il simule une viscosité, une raideur ou une inertie (ou une combinaison) appliquée à l'instrument 20 et que doit ressentir l'opérateur.

In fine, le dispositif applique des forces à l'instrument 20 au niveau du point d'attache P.

La position et/ou la vitesse observée sur l'instrument 20 sont envoyées à l'unité de traitement U qui en retour envoie un effort à appliquer (voir **figure 2**)

Les impédances applicables dépendent de plusieurs paramètres, comme du mode d'utilisation ou du geste qu'est en train d'effectuer l'opérateur. Une viscosité $\mu$ est une impédance qui relie un effort à une vitesse :

$$\vec{F} = -\mu\vec{v}$$

Une raideur k est une impédance qui relie un effort à un écart par rapport à une position de référence (selon le signe la raideur, on aura une force de rappel ou de répulsion) :

$$\vec{F} = k(\vec{x}_0 - \vec{x})$$

Une masse m est une impédance qui relie un effort à une accélération :

$$\vec{F} = -m\frac{d\vec{v}}{dt}$$

Il existe différentes lois préétablies, qui déterminent quelles valeurs d'impédance appliquer. Ces impédances peuvent elles-mêmes être fonction de la position ou de la vitesse d'un point de l'instrument 20.

Comme mentionné en introduction, le point d'attache P qui correspond directement au point d'application de l'effort du dispositif 1 vers l'instrument 20 semble le point naturel pour appliquer une impédance. Or, pour des raisons d'amélioration de l'utilisation du dispositif 1, on peut par exemple décider que la raideur ressentie par l'opérateur soit la même au niveau de la poignée H, quelle que soit la position du point d'appui F sur l'instrument 20, c'est-à-dire quel que soit l'enfoncement de l'instrument 20 dans le corps du patient.

Par conséquent, il faut pouvoir définir la viscosité quasiment à chaque instant au point relatif à la poignée H.

Un autre exemple peut être pris : on peut vouloir définir la viscosité au niveau de la pointe T.

Dans tous ces cas, pour appliquer un effort qui soit pertinent et aide l'opérateur à manipuler l'instrument 20, il est nécessaire d'obtenir des données relatives à la position ou à la vitesse de ces points (H ou T ou un autre point de l'axe d'instrument XX'), qui sont différents du point d'attache P.

<u>Méthode du bras de levier</u>

Un des procédés proposés permet d'obtenir ces données pour un point Q quelconque situé sur l'axe d'instrument X-X'. Pour cela, on suppose que l'instrument 20 est mis en place dans la canule 30 et possède ledit point d'appui F. Par conséquent, l'axe d'instrument X-X' passe par les points d'attache P et d'appui F.

On suppose aussi que la position du point d'appui F est connue. Il existe plusieurs méthodes pour connaître cette position. On peut par exemple effectuer une routine de calibrage au préalable, ou entrer les coordonnées, ou bien appliquer une méthode qui sera décrite par la suite.

Le procédé pour obtenir lesdites données d'un point situé sur l'axe d'instrument X-X' consiste à effectuer les étapes suivantes, au moyen de l'unité de traitement U :

- déterminer E1 des données relatives à une position et/ou une vitesse du point d'attache P dans le référentiel lié au dispositif 1,
- déterminer E2 des données de vitesse ou de position d'un point Q de l'instrument 20 situé sur l'axe d'instrument X-X' à l'aide desdites données relatives au point d'attache P, de la distance connue $\overline{PQ}$ du point d'attache P au point quelconque Q sur l'axe d'instrument X-X', et de la position du point d'appui F, que l'on connaît,
- déterminer E3 un effort $\overrightarrow{F_Q}$ à l'aide d'une impédance à conférer à l'instrument au niveau dudit point Q de l'instrument 20, et à l'aide des données déterminées à l'étape E2 dudit point Q de l'instrument 20,
- déterminer E4 un effort $\overrightarrow{F_P}$ à appliquer au point d'attache P à l'aide de l'effort $\overrightarrow{F_Q}$ précédent au niveau du point quelconque Q, et à l'aide des données relatives au point d'attache P et de la position du point d'appui F,
- piloter E5 des motorisations M1, M2, M3 pour transmettre l'effort $\overrightarrow{F_P}$ à appliquer au point d'attache P à l'instrument au niveau du point d'attache P.

L'étape E1 comprend ainsi une acquisition de données issues des capteurs avec un traitement par l'unité de traitement U, les étapes E2 à E4 sont des étapes de traitement par l'unité de traitement U, et enfin l'étape E5 comprend des consignes d'activation des motorisations.

Un tel procédé ne nécessite pas de connaître l'orientation de l'axe d'instrument 20 et par conséquent ne nécessite pas d'utiliser les capteurs angulaires C4, C5 au niveau de la liaison rotule du point d'attache P.

Comme mentionné précédemment, de tels capteurs sont de mauvaise qualité, à l'inverse des capteurs C1, C2, C3 des articulations A1, A2, A3, qui offrent un signal précis avec peu de bruit.

Au cours de l'étape E2, pour déterminer la position du point Q à partir de la position du point P, de la position du point F, et de la distance $d = \overline{PQ}$ de P à Q le long de l'axe d'instrument X-X', on peut procéder de la façon suivante : calculer d'abord le vecteur unitaire $\vec{x}_I$ de l'axe d'instrument X-X' :

$$\vec{x}_I = \left(1/\left\|\overrightarrow{PF}\right\|\right)\overrightarrow{PF} \text{ ,}$$

puis calculer la position du point Q par rapport au point P :

$$\overrightarrow{PQ} = d\,\vec{x}_I \text{ .}$$

Au cours de la même étape E2, connaître la position du point P, la vitesse du point P, la position de F, et $\vec{x}_I$ permet de calculer la vitesse d'un point donné Q de l'axe d'instrument situé à une distance donnée $d = \overline{PQ}$ de P, grâce à un modèle de levier connu de l'homme de l'art :

$$\vec{v}(Q) = (\vec{v}(P).\vec{z}_I)\vec{z}_I + \left(\frac{\overline{FQ}}{\overline{FP}}\right)(\vec{v}(P) - (\vec{v}(P).\vec{z}_I)\vec{z}_I) \text{ ,}$$

où $\overline{FP}$ et $\overline{FQ} = \overline{FP} + d$ et sont les distances signées des points F à P, et de F à Q, respectivement.

De façon duale, lors de l'étape E4, connaître la force $\overrightarrow{F_Q}$ qu'il faudrait appliquer au point Q permet, grâce à un modèle de levier connu de l'homme de l'art d'appliquer la force équivalente à appliquer au point P :

$$\vec{F}_P = (\vec{F}_Q.\vec{x}_I)\vec{x}_I + \left(\frac{\overline{FQ}}{\overline{FP}}\right)(\vec{F}_Q - (\vec{F}_Q.\vec{x}_I)\vec{x}_I)$$

Pour un mouvement de rotation pure autour du point d'appui F, la connaissance du bras de levier et d'une vitesse d'un seul point (le point P) suffit (voir **figure 5a**). Pour un mouvement de translation le long de l'axe X-X', tous les points de l'instrument 20 ont la même vitesse (voir **figure 5b**).

La **figure 3** présente les distances qui interviennent dans les calculs, notamment les longueurs $l_{HP}$, $l_{PF}$ et $l_{PT}$ si le point Q correspond à la poignée ou à la pointe.

Une fois obtenu l'effort qu'il faudrait appliquer au point quelconque Q, le bras de levier permet de remonter à l'effort que doivent appliquer les motorisations M1, M2, M3 au niveau du point d'attache P.

On obtient la chaîne logique suivante, lorsque par exemple l'impédance ne fait intervenir que la vitesse :

$$\vec{v}(P) \xRightarrow{E2\text{ (levier)}} \vec{v}(Q) \xRightarrow{E3\text{ (impédance en Q)}} \overrightarrow{F_Q} \xRightarrow{E4\text{ (levier)}} \overrightarrow{F_P}$$

Selon un mode préférentiel, le point Q correspond au point T relatif à la pointe, ou au point H relatif à la poignée.

Ceci présente un intérêt dans la qualité de l'interaction. Par exemple, si l'impédance est un simple coefficient de viscosité et que le point quelconque Q coïncide avec le point T, alors on obtiendra par ce procédé une viscosité isotrope au point T, c'est-à-dire que la force en T, $\overrightarrow{F_T}$, sera toujours parallèle à la vitesse de T, $\vec{v}(T)$ à laquelle elle s'oppose.

Au contraire, si le point quelconque Q était coïncidant avec le point P, comme il est classique de procéder sans faire appel aux étapes (E2) et (E4), alors on aurait une viscosité anisotrope au point T, c'est-à-dire que la force en T , $\overrightarrow{F_T}$, ne serait pas nécessairement parallèle à la vitesse de T, $\vec{v}(T)$.

Par ailleurs, les calculs détaillés en trois étapes ci-dessus peuvent s'appliquer, après reformulation, en une seule étape ne faisant pas apparaître explicitement la vitesse de Q $\vec{v}(Q)$ et la force à appliquer au point quelconque Q, ce qui revient à écrire une fonction directe reliant la position et la vitesse du point d'attache P à la force à appliquer au point d'attache P, en exploitant uniquement la connaissance de la position de F, l'impédance à appliquer au point quelconque Q, et la distance d définissant la position connue du point Q sur l'axe d'instrument X-X'. Par conséquent, les étapes E2 à E4 peuvent être réduites à étape de traitement dans laquelle on détermine un effort à appliquer au point d'attache en fonction desdites données relatives au point d'attache, et de distance connue du point d'attache P au point quelconque Q, dans le but de conférer une impédance donnée au point quelconque Q.

Méthode d'auto-calibrage

La méthode d'auto-calibrage permet tout d'abord de savoir si l'instrument 20 est effectivement positionné dans une canule 30 et possède donc un point d'appui F, et, le cas échéant, de connaître la position dudit point d'appui F.
On suppose que l'unité de traitement U peut connaître l'équation de l'axe d'instrument. Cela est rendu possible grâce aux cinq capteurs du bras articulé C1, C2, C3, C4, C5.
A nouveau, on rappelle qu'il n'est pas nécessaire d'avoir de sixième capteur.
Le procédé comprend les étapes suivantes :

- Obtenir E01 dans le référentiel du dispositif d'une pluralité de droites $\Delta$ définies par l'axe d'instrument X-X', les droites correspondant à une pluralité de configurations de l'instrument,
- estimer E02 l'existence d'une zone $V_{ol}$ d'intersection de ladite pluralité de droites $\Delta$,
- obtention E03 de la position centrale de ladite zone $V_{ol}$ si elle existe, ladite zone correspondant alors au point d'appui de l'instrument F.

Dans cette méthode, il n'est pas nécessaire de disposer de motorisations M1, M2, M3. En effet, pour connaître lesdites droites, il suffit de disposer des capteurs suffisants, dans le cas présent les cinq capteurs C1 à C5.
La **figure 6** représente en superposition des droites issues d'un déplacement dudit instrument 20. Comme illustré sur cette figure, elles se recoupent en une zone correspondant au point d'appui F.
En effet, l'imprécision des capteurs angulaires C4, C5 est compensée par la pluralité de mesures effectuées, soit grâce à une moyenne ou un filtrage, ou les deux. La durée d'acquisition est de l'ordre de la milliseconde, ce qui signifie qu'environ en une seconde, une quantité d'information suffisante est rassemblée pour obtenir un résultat fiable quant à l'existence ou non d'une zone d'intersection et sa position éventuelle.
Des algorithmes de résolution de système matriciel linéaire sont connus dans la littérature. En particulier, à cause des imprécisions de mesures et l'immobilité non parfaite de la canule 30, la zone d'intersection est un volume $V_{ol}$. En fonction des critères choisis (taille, etc.), on peut valider ou non la présence d'un point d'appui.
Par exemple, la résolution du système linéaire peut se faire par une approche des moindres carrés.
La position du point d'appui F correspond par exemple au centre d'un tel volume $V_{ol}$.
D'une façon pratique, lorsque l'opérateur saisit l'instrument, l'unité U calcule à intervalle régulier l'équation de la droite $\Delta$ de l'axe d'instrument X-X' (toutes les millisecondes par exemple). L'unité U peut également attendre, avant de calculer une nouvelle équation de droite, non pas un temps donné, mais un déplacement donné du point P pour s'assurer que toutes les droites ne seront pas superposées. Tant que l'opérateur n'a pas inséré l'instrument 20 dans une canule 30, l'unité U ne trouvera pas de zone d'intersection et par conséquent saura que l'instrument 20 n'est pas inséré dans une canule 30.
Une fois que l'opérateur a introduit l'instrument 20 dans une canule 30, l'unité U détermine, à une échéance de l'ordre d'une seconde, l'existence d'une telle zone et connaît ainsi la position du point d'appui F.
Le procédé peut comprendre une étape supplémentaire E05 d'application d'un effort au niveau du point d'appui P à l'aide impédance prédéterminée, lorsqu'aucune zone d'intersection n'est identifiée.
On peut par exemple appliquer une viscosité assez faible au niveau du point d'attache P de façon à ce que l'opérateur déplace l'instrument 20 aisément.
Ce procédé de détection automatique du point d'appui F, contrairement aux routines préexistantes, ne nécessite pas d'être effectué préalablement. L'opérateur peut ainsi directement utiliser l'instrument 20.
Cela fournit des avantages importants :

- lorsque le patient bouge, ou lorsque l'opérateur fait bouger le patient (choc dans la table d'opération), la position du point d'appui F change et le dispositif 1 peut donc en alerter l'opérateur,
- lorsque l'opérateur change de canule 30, il n'a pas besoin d'effectuer à nouveau un calibrage, d'où un gain de temps et une baisse des risques.

Un tel procédé peut être exploité indépendamment de la méthode du bras de levier décrite précédemment.

Méthode avec modèle de viscosité filtrée

Cette méthode concerne les cas où l'impédance est une viscosité et que l'on souhaite éviter les effets d'instabilité mentionnés en introduction (voir courbe 70 sur la **figure 4**, par rapport à la courbe 71 de référence).
On suppose que l'on connaît la position d'un point Q (pour cela, on peut si nécessaire utiliser le procédé susmentionné lors de la description de la méthode du bras de levier).
Le procédé comprend les étapes suivantes :

- (E1') déterminer la vitesse instantanée $\vec{v}(Q)$ d'un point Q de l'instrument 20 dans le référentiel lié au dispositif d'assistance 1,
- (E21') déterminer une première viscosité fonction décroissante de ladite vitesse instantanée $\vec{v}(Q)$,
- (E22') déterminer une deuxième viscosité à partir de la première viscosité grâce à un procédé de filtrage possédant au moins un paramètre permettant de régler la dynamique du procédé,
- (E3') déterminer un effort $\overrightarrow{F_Q}$ au niveau dudit point Q de l'instrument 20, fonction:

  ◦ de ladite vitesse $\vec{v}(Q)$,
  ◦ de la deuxième valeur de viscosité,

Enfin, l'étape de pilotage des motorisations est classique.
Lorsque le point Q est différent du point d'attache P, le modèle du bras de levier peut être utilisé à la fois pour le calcul des vitesses ($\vec{v}(P) \Rightarrow \vec{v}(Q)$) et pour le calcul des forces ($\vec{F}_Q \Rightarrow \vec{F}_P$).
Lorsque le signal de vitesse $\vec{v}(Q)$ est bruité, on peut ajouter une étape de filtrage de ladite vitesse $\vec{v}(Q)$ entre l'étape E1' et l'étape E21'.
Un tel procédé ralentit la dynamique de variation de la viscosité et offre une stabilité non permise précédemment (voir courbe 72 sur la **figure 4**).
Le coefficient paramétrable est typiquement une constante de temps que l'on peut ajuster pour optimiser la dynamique du procédé.
Un tel procédé peut être exploité indépendamment des méthodes du bras de levier et d'auto-calibrage décrites précédemment.

Méthode de l'instrument pointeur

Un autre procédé va à présent être décrit. De la même façon, il peut être avantageusement appliqué en combinaison du procédé permettant d'appliquer un effort au niveau du point d'attache P.
Par exemple, on souhaite établir une contrainte géométrique grâce à des champs de force élastiques, comme un plan d'attraction ou de répulsion pour établir un guide pour l'instrument 20. Par exemple, si une zone ne doit pas être atteinte dans le patient, pouvoir définir un plan de répulsion permet de limiter les risques pour l'opérateur.
Plus généralement, pour établir la contrainte, on définit un point d'intérêt de l'instrument : ce point d'intérêt est avantageusement son extrémité distale.
Pour cela, le procédé comprend les étapes suivantes :

- faire coïncider E01' le point d'intérêt avec des points de l'espace et déterminer E02' leur position dans le référentiel lié au dispositif d'assistance 1,
- construire E03' une contrainte géométrique définie par lesdits points de l'espace à l'aide desdites positions.

Lorsque le point d'intérêt est l'extrémité distale, le procédé consiste pour l'opérateur à désigner dans l'espace des points avec ladite extrémité.
Une fois cela effectué, on peut définir plusieurs types de contraintes géométriques.
Par exemple, la contrainte géométrique peut être un plan et dans ce cas il est avantageux de pointer trois points non coplanaires, le plan étant ensuite déterminé comme celui qui passe par lesdits trois points.
La contrainte géométrique peut aussi être une droite et dans ce cas il est avantageux de pointer deux points distincts, la droite étant ensuite déterminée comme celle qui passe par lesdits deux points.
La contrainte géométrique peut être une sphère et dans ce cas il est avantageux de pointer deux points distincts, la sphère étant ensuite définie comme étant celle dont le centre est le premier desdits deux points et qui passe par le second desdits deux points.
La contrainte peut être réduite à un seul point et dans ce cas il est avantageux de la définir en pointant directement ce point.
En **figure 7** sont représentés plusieurs plans PLAN1,..., PLAN 5, qui définissent un espace dans lequel l'instrument est incité à rester (plans de répulsion), en utilisant des raideurs appropriées pour chacun des plans PLAN1, ..., PLAN 5.
Dans cet exemple, le point d'intérêt défini pour la méthode du pointage et le point quelconque Q défini pour la méthode du bras de levier sont un même et unique point. Le procédé comprend alors avantageusement les étapes suivantes, suite à la détermination du plan :

- détermination E31' de la distance entre ledit point Q de l'instrument 20 et le plan PLAN1, par projection orthogonale,

- détermination E32" de l'effort $\overrightarrow{F_Q}$ au niveau dudit point Q, ledit effort $\overrightarrow{F_Q}$ étant fonction d'un coefficient de raideur et de ladite distance.

Les étapes de détermination de l'effort E4 au niveau du point d'attache P et de pilotage E5 des motorisations M1, M2, M3 sont celles classiquement utilisés ou celles précédemment décrites.

Méthode du changement d'état

Cette méthode permet d'améliorer le confort et l'intuitivité d'utilisation de dispositif pour l'opérateur.
Pour cela, l'unité de traitement U a été configurée pour comprendre plusieurs modes de commande, chaque mode de commande ayant une impédance prédéterminée et un critère de commutation prédéterminé. Le procédé consiste à commuter entre des modes lorsqu'un critère de commutation prédéterminé est vérifié.
On obtient ainsi une commutation automatique ne nécessitant pas d'action autre que la manipulation de l'instrument par l'opérateur. Avantageusement, les critères prédéterminés ne dépendent que des mesures fournies par le bras articulé.
La méthode du changement d'état peut s'appliquer à n'importe quel moment lors des autres méthodes décrites.
Selon un mode de réalisation, le procédé consiste à changer d'état entre deux modes de commande appelés mode verrouillé (destiné à maintenir l'instrument en position même si l'opérateur le lâche) et mode libre (destiné à laisser l'opérateur libre de manipuler l'instrument).
Le procédé peut alors comprendre les étapes suivantes :

- vérification E6 d'un critère de verrouillage et mise en mode verrouillé pour lequel une impédance de verrouillage prédéterminée est appliquée à l'instrument, si la vérification est positive,
- vérification E7 d'un critère de déverrouillage et mise en mode libre pour lequel une impédance de liberté prédéterminée est appliquée à l'instrument, si la vérification est positive.

Un mode de réalisation avantageux définit l'impédance de liberté comme une viscosité de faible valeur pour permettre sa manipulation par l'opérateur et/ou l'impédance de verrouillage comprend une raideur suffisamment grande pour garantit le maintien en position de l'instrument.
Les autres méthodes décrites précédemment, peuvent ensuite s'appliquer.
Un mode de réalisation avantageux définit le critère de verrouillage comme une immobilité pendant une durée déterminée (par exemple trois secondes) et le critère de déverrouillage comme une translation « sortante » de l'instrument selon l'axe d'instrument X-X' (voir **figure 8a**). Ces deux critères sont indépendants.
En **figures 8b et 8c** sont représentés deux mouvements qui, dans le cas présent, ne déverrouilleraient pas le mode verrouillé.

**Revendications**

1. Dispositif d'assistance à la manipulation d'un instrument (20), comprenant :
un bras articulé (10) destiné à être fixé à un bâti (B) et manipulable par un opérateur, sur lequel un instrument (20) peut être fixé au niveau d'un point d'attache (P) dudit bras articulé (10) formant une liaison rotule passive entre le bras articulé (10) et l'instrument (20), la liaison rotule passive ne transmettant pas de moment au point d'attache (P) de l'instrument (20) et permet une manipulation libre de l'instrument (20) par l'opérateur, le bras articulé comprenant des motorisations (M1, M2, ...) pour déplacer le point d'attache (P) dans un référentiel (R) lié au bâti (B), l'instrument (20) étant manipulable autour d'un point d'appui (F) ayant une position connue et fixe dans le référentiel (R),
une unité de traitement (U) comprenant un processeur (U1) configurée pour piloter les motorisations (M1, M2, ...) ;le dispositif étant configuré pour mettre en oeuvre une étape (E0) préliminaire de détermination de la position du point d'appui (F) dans ledit référentiel (R) de la manière suivante :

   - Obtention (E01), dans le référentiel (R) d'une pluralité de droites (Δ) définies par l'axe d'instrument (X-X') les droites correspondant à une pluralité de positions dudit instrument (20) autour du point d'appui (F) et passent pas le point d'attache (P), chaque droite étant obtenue après un déplacement donné du point d'attache (P) ;
   - Estimation (E02) d'une zone ($V_{ol}$) d'intersection de ladite pluralité de droites (Δ),
   - Obtention (E03) de la position du centre de ladite zone ($V_{ol}$), qui correspond au point d'appui (F),

le dispositif étant configuré pour déterminer (E1) des données relatives à une position et/ou une vitesse du point d'attache (P) dans le référentiel (R);
l'unité de traitement étant configurée pour :

déterminer (E2, E3, E4) un effort ($\overrightarrow{F_P}$) à appliquer au point d'attache (P) de manière à appliquer une impédance donnée en un point (Q) situé sur l'axe (X-X') d'instrument reliant le point d'attache (P) au point d'appui (F), le point (Q) étant situé à une distance connue ($\overrightarrow{PQ}$) du point d'attache (P), l'effort à appliquer étant fonction desdites données relatives au point d'attache (P), de la position du point d'appui (F), de la distance connue ($\overrightarrow{PQ}$) du point d'attache (P) au point (Q), et de l'impédance donnée à appliquer au point (Q);
le dispositif étant en outre configuré pour piloter (E5) des motorisations (M1, M2,...) pour transmettre l'effort ($\overrightarrow{F_P}$) déterminé à l'instrument (20) au niveau du point d'attache (P), de manière à produire l'impédance au point (Q).

**2.** Dispositif selon la revendication précédente, dans lequel l'unité de traitement (U) est configurée pour mettre en oeuvre l'étape de détermination de l'effort ($\overrightarrow{F_P}$) à appliquer au point d'attache de la manière suivante :

- déterminer (E2) des données de vitesse et/ou de position du point (Q) de l'instrument situé sur un axe d'instrument (X-X') passant par le point d'attache (P) et le point d'appui (F), au moyen desdites données relatives au point d'attache (P) et de la position du point d'appui (F);
- déterminer (E3) un effort ($\overrightarrow{F_Q}$) à appliquer au point Q fonction d'une impédance à conférer à l'instrument (20) au niveau dudit point (Q) de l'instrument (20) et fonction des données déterminées dudit point (Q) de l'instrument (20);
- déterminer (E4) un effort ($\overrightarrow{F_P}$) à appliquer au point d'attache (P) fonction de l'effort ($\overrightarrow{F_Q}$) à appliquer au point (Q) et des données relatives au point d'attache P et de la position du point d'appui (F).

**3.** Dispositif selon l'une quelconque des revendications précédentes, configuré pour que l'impédance comprenne une viscosité et l'effort ($\overrightarrow{F_P}$) appliqué au point d'attache (P) est une force de résistance dans le sens contraire à son déplacement.

**4.** Dispositif selon l'une quelconque des revendications précédentes, configuré pour que l'impédance comprenne une raideur et l'effort ($\overrightarrow{F_P}$) appliqué au point d'attache (P) est une force dans le même sens ou le sens contraire à son déplacement, selon la signe de la raideur.

**5.** Dispositif selon la revendication précédente, dans lequel le bras articulé (10) comprend trois articulations (A1, A2, A3) chacune actionnée par une motorisation (M1, M2, M3) et comprenant chacune un capteur (C1, C2, C3) de manière à déterminer la position du point d'attache (P) dans le référentiel (R).

**6.** Dispositif selon l'une des revendications précédentes, dans lequel le dispositif (1) comprend deux capteurs de position angulaire (C4, C5) au niveau du point d'attache (P) pour obtenir l'orientation de l'axe d'instrument (X-X').

**7.** Dispositif selon l'une quelconque des revendications précédentes, dans lequel l'unité de traitement (U) est configurée pour que l'impédance comprenne une viscosité, et dans lequel l'étape (E3) de détermination de l'effort (Fq) au niveau dudit point (Q) est mise en oeuvre de la manière suivante pour déterminer l'impédance à conférer :

- détermination (E1') de la vitesse instantanée ($\vec{v}(Q)$) dudit point (Q) de l'instrument,
- détermination (E21') d'une première viscosité fonction décroissante de ladite vitesse instantanée ($\vec{v}(Q)$),
- détermination (E22') d'une deuxième viscosité à partir de la première viscosité grâce à un procédé de filtrage possédant au moins un paramètre permettant de régler la dynamique du procédé ;
- détermination (E3') de l'effort au niveau dudit point de l'instrument, en fonction :

◦ de ladite vitesse ($\bar{v}(Q)$), et
◦ de la deuxième valeur de viscosité permettant d'ajuster la dynamique.

**8.** Dispositif selon la revendication précédente, dans lequel l'unité de traitement (U) est configurée pour que la vitesse instantanée du point (Q) soit filtrée.

**9.** Dispositif selon l'une quelconque des revendications précédentes, dans lequel l'unité de traitement (U) est configurée, en outre, pour mettre en oeuvre des étapes consistant à :

- faire coïncider (E01') un point de l'instrument avec des points de l'espace et déterminer la position desdits points de l'espace dans le référentiel lié au dispositif d'assistance ;
- construire (E03') une contrainte géométrique définie par lesdits points de l'espace à l'aide desdites positions.

**10.** Dispositif selon la revendication précédente, dans lequel l'unité de traitement (U) est configurée pour que l'impédance comprenne une raideur, et dans lequel l'unité de traitement est configurée pour mettre en oeuvre une étape préliminaire de construction d'un plan, ledit point d'instrument étant une extrémité distale, ladite étape comprenant la sous-étape suivante :

- Pointage (E01') avec l'extrémité distale d'au moins trois points non colinéaires et détermination de leur position, et détermination (E02') de leur position dans le référentiel (R),
- Construction (E03') d'un plan (PLAN1) passant par les trois points à l'aide des trois positions,

l'étape (E3) de détermination de l'effort ($\overrightarrow{F_P}$) au niveau dupoint d'attache (P) comprenant les sous-étapes suivantes:

- détermination (E31") de la distance entre ledit point (Q) de l'instrument (20) et le plan par projection orthogonale,
- détermination (E32") de l'effort ($\overrightarrow{F_Q}$) fonction d'une raideur et de ladite distance.

de sorte que l'étape de pilotage (E5) des motorisations contraint l'instrument à se positionner par rapport au plan (PLAN1) en provoquant l'attraction ou la répulsion dudit point (Q) de l'axe d'instrument (X-X') par rapport audit plan (PLAN1).

**11.** Dispositif selon l'une quelconque des revendications précédentes, dans lequel une pluralité de modes de commutation est implémentée dans l'unité de traitement (U), chaque mode ayant une impédance prédéterminée, l'unité de traitement (U) étant configurée pour mettre en oeuvre des étapes consistant à commuter automatiquement d'un mode de commande à un autre lorsqu'un critère est vérifié.

**12.** Dispositif selon la revendication précédente, dans lequel ledit au moins un critère ne fait intervenir que des données relatives audit dispositif d'assistance lors de la mise oeuvre du procédé.

**13.** Dispositif selon l'une quelconque des revendications 12 ou 13, dans lequel l'unité de traitement (U) est configurée pour mettre en oeuvre deux impédances qui sont paramétrées pour définir respectivement un mode libre et un mode verrouillée, et met en oeuvre un critère de verrouillage qui permet de passer du mode libre au mode verrouillé et un critère de déverrouillage qui permet de passer du mode verrouillé au mode libre.

**14.** Dispositif selon la revendication précédente dans lequel le critère de verrouillage est une immobilité de l'instrument (20) pendant une durée déterminée et le critère de déverrouillage est une translation de l'instrument (20) selon l'axe d'instrument (X-X').

## Patentansprüche

**1.** Vorrichtung zur Unterstützung der Handhabung eines Instruments (20), umfassend:

einen Gelenkarm (10), der zur Befestigung an einem Gestell (B) bestimmt und von einem Bediener handhabbar ist, an dem ein Instrument (20) im Bereich eines Befestigungspunktes (P) des Gelenkarms (10) befestigbar ist, der eine passive Kugelgelenkverbindung zwischen dem Gelenkarm (10) und dem Instrument (20) bildet, wobei die passive Kugelgelenkverbindung kein Moment an den Befestigungspunkt (P) des Instruments (20) überträgt und eine freie Handhabung des Instruments (20) durch den Bediener gestattet, wobei der Gelenkarm Motorisierungen (M1, M2, ...) zur Verlagerung des Befestigungspunktes (P) in einem Bezugssystem (R) umfasst, das mit dem Gestell (B) verbunden ist, wobei das Instrument (20) um einen Auflagepunkt (F) handhabbar ist, der eine bekannte und feste Position in dem Bezugssystem (R) hat,
eine Verarbeitungseinheit (U), die einen Prozessor (U1) umfasst, der dafür ausgelegt ist, die Motorisierungen

(M1, M2, ...) zu steuern; wobei die Vorrichtung dafür ausgelegt ist, einen vorbereitenden Schritt (E0) des Bestimmens der Position des Auflagepunktes (F) im Bezugssystem (R) folgendermaßen durchzuführen:

- Erhalten (E01), in dem Bezugssystem (R), einer Vielzahl von Geraden ($\Delta$), die von der Instrumentenachse (X-X') bestimmt sind, wobei die Geraden einer Vielzahl von Positionen des Instruments (20) um den Auflagepunkt (F) entsprechen und durch den Auflagepunkt (P) verlaufen, wobei jede Gerade nach einer gegebenen Verlagerung des Auflagepunktes (P) erhalten wird;
- Schätzen (E02) einer Schnittpunktzone ($V_{0l}$) der Vielzahl von Geraden ($\Delta$),
- Erhalten (E03) der Position des Zentrums der Zone ($V_{0l}$), die dem Auflagepunkt (F) entspricht,

wobei die Vorrichtung dafür ausgelegt ist, Daten zu bestimmen (E1), die sich auf eine Position und/oder eine Geschwindigkeit des Befestigungspunktes (P) in dem Bezugssystem (R) beziehen;
wobei die Verarbeitungseinheit ausgelegt ist, um:

eine Kraft ($\overrightarrow{F_P}$) zu bestimmen (E2, E3, E4), die auf den Befestigungspunkt (P) derart anzuwenden ist, dass eine gegebene Impedanz in einem Punkt (Q) angewendet wird, der sich auf der Instrumentenachse (X-X') befindet, die den Befestigungspunkt (P) mit dem Auflagepunkt (F) verbindet, wobei sich der Punkt (Q) in einem bekannten Abstand ($\overrightarrow{PQ}$) vom Befestigungspunkt (P) befindet, wobei die anzuwendende Kraft von den Daten, die sich auf den Befestigungspunkt (P) beziehen, von der Position des Auflagepunktes (F), von dem bekannten Abstand ($\overrightarrow{PQ}$) des Befestigungspunktes (P) zum Punkt (Q) und von der gegebenen Impedanz, die auf den Punkt (Q) anzuwenden ist, abhängt,
wobei die Vorrichtung ferner dafür ausgelegt ist, Motorisierungen (M1, M2, ...) zu steuern (E5), um die bestimmte Kraft ($\overrightarrow{F_P}$) an das Instrument (20) im Bereich des Befestigungspunktes (P) derart zu übertragen, dass die Impedanz am Punkt (Q) erzeugt wird.

**2.** Vorrichtung nach vorangehendem Anspruch, wobei die Verarbeitungseinheit (U) dafür ausgelegt ist, den Schritt des Bestimmens der am Befestigungspunkt anzuwendenden Kraft ($\overrightarrow{F_P}$) folgendermaßen durchzuführen:

- Bestimmen (E2) der Geschwindigkeits- und/oder Positionsdaten des Punktes (Q) des Instruments, der sich auf einer Instrumentenachse (X-X') befindet, die durch den Befestigungspunkt (P) und den Auflagepunkt (F) verläuft, mittels der Daten, die sich auf den Befestigungspunkt (P) beziehen und der Position des Auflagepunktes (F);
- Bestimmen (E3) einer Kraft ($\overrightarrow{F_Q}$), die auf den Punkt Q als Funktion einer Impedanz, die dem Instrument (20) im Bereich des Punktes (Q) des Instruments (20) zu verleihen ist und als Funktion der bestimmten Daten des Punktes (Q) des Instruments (20) anzuwenden ist;
- Bestimmen (E4) einer Kraft ($\overrightarrow{F_P}$), die auf den Befestigungspunkt (P) als Funktion der Kraft ($\overrightarrow{F_Q}$), die auf den Punkt (Q) anzuwenden ist, und der Daten, die sich auf den Befestigungspunkt P beziehen, und der Position des Auflagepunktes (F) anzuwenden ist.

**3.** Vorrichtung nach einem der vorangehenden Ansprüche, die ausgelegt ist, damit die Impedanz eine Viskosität umfasst und die am Befestigungspunkt (P) angewendete Kraft ($\overrightarrow{F_P}$) eine Widerstandskraft in der zu seiner Verlagerung entgegengesetzten Richtung ist.

**4.** Vorrichtung nach einem der vorangehenden Ansprüche, die ausgelegt ist, damit die Impedanz eine Steifheit umfasst und die am Befestigungspunkt (P) angewendete Kraft ($\overrightarrow{F_P}$) je nach dem Vorzeichen der Steifheit eine Kraft in der zu seiner Verlagerung derselben Richtung oder entgegengesetzten Richtung ist.

**5.** Vorrichtung nach vorangehendem Anspruch, wobei der Gelenkarm (10) drei Gelenke (A1, A2, A3) umfasst, die jeweils von einer Motorisierung (M1, M2, M3) betätigt werden und jeweils einen Sensor (C1, C2, C3) umfassen, um die Position des Befestigungspunktes (P) im Bezugssystem (R) zu bestimmen.

**6.** Vorrichtung nach einem der vorangehenden Ansprüche, wobei die Vorrichtung (1) zwei Winkelpositionssensoren (C4, C5) im Bereich des Befestigungspunktes (P) umfasst, um die Ausrichtung der Instrumentenachse (X-X') zu erhalten.

**7.** Vorrichtung nach einem der vorangehenden Ansprüche, wobei die Verarbeitungseinheit (U) ausgelegt ist, damit

die Impedanz eine Viskosität umfasst, und wobei der Schritt (E3) zum Bestimmen der Kraft (Fq) im Bereich des Punktes (Q) folgendermaßen durchgeführt wird, um die zu verleihende Impedanz zu bestimmen:

- Bestimmen (E1') der Momentangeschwindigkeit ($\vec{v}(Q)$) des Punktes (Q) des Instruments,
- Bestimmen (E21') einer ersten Viskosität als abnehmende Funktion der Momentangeschwindigkeit ($\vec{v}(Q)$),
- Bestimmen (E22') einer zweiten Viskosität ausgehend von der ersten Viskosität dank eines Filterverfahrens, das mindestens einen Parameter besitzt, der gestattet, die Dynamik des Verfahrens zu regeln;
- Bestimmen (E3') der Kraft im Bereich des Punktes des Instruments in Abhängigkeit:

    o von der Geschwindigkeit ($\vec{v}(Q)$), und
    o des zweiten Viskositätswertes, der gestattet, die Dynamik anzupassen.

**8.** Vorrichtung nach vorangehendem Anspruch, wobei die Verarbeitungseinheit (U) ausgelegt ist, damit die Momentangeschwindigkeit des Punktes (Q) gefiltert wird.

**9.** Vorrichtung nach einem der vorangehenden Ansprüche, wobei die Verarbeitungseinheit (U) ferner ausgelegt ist, um die Schritte durchzuführen, die darin bestehen:

- einen Punkt des Instruments mit Punkten des Raums in Übereinstimmung zu bringen (E01') und die Position der Punkte des Raums in dem Bezugssystem, das mit der Unterstützungsvorrichtung verbunden ist, zu bestimmen;
- eine von den Raumpunkten definierten geometrische Beanspruchung mit Hilfe der Positionen zu konstruieren (E03').

**10.** Vorrichtung nach vorangehendem Anspruch, wobei die Verarbeitungseinheit (U) ausgelegt ist, damit die Impedanz eine Steifigkeit umfasst, und wobei die Verarbeitungseinheit ausgelegt ist, um einen vorbereitenden Konstruktionsschritt einer Ebene durchzuführen, wobei der Instrumentenpunkt ein distales Ende ist, wobei der Schritt den folgenden Unterschritt umfasst:

- Ansteuern (E01'), mit dem distalen Ende, von mindestens drei nicht-kolinearen Punkten und Bestimmen ihrer Position, und Bestimmen (E02') ihrer Position im Bezugssystem (R),
- Konstruieren (E03') einer Ebene (PLAN1), die durch die drei Punkte verläuft, mit Hilfe der drei Positionen, wobei der Schritt (E3) des Bestimmens der Kraft ($\overrightarrow{F_P}$) im Bereich des Befestigungspunktes (P) die folgenden Unterschritte umfasst:

    - Bestimmen (E31") des Abstands zwischen dem Punkt (Q) des Instruments (20) und der Ebene durch orthogonale Projektion,
    - Bestimmen (E32") der Kraft ($\overrightarrow{F_Q}$) als Funktion einer Steifigkeit und des Abstands,

so dass der Schritt des Steuerns (E5) der Motorisierungen das Instrument zwingt, sich im Verhältnis zur Ebene (PLAN1) durch Anziehung oder Abstoßung des Punktes (Q) der Instrumentenachse (X-X') im Verhältnis zur Ebene (PLAN1) zu positionieren.

**11.** Vorrichtung nach einem der vorangehenden Ansprüche, wobei eine Vielzahl von Schaltmodi in der Verarbeitungseinheit (U) implementiert ist, wobei jeder Modus eine vorbestimmte Impedanz hat, wobei die Verarbeitungseinheit (U) ausgelegt ist, um die Schritte durchzuführen, die darin bestehen, von einem Steuermodus automatisch in einen anderen zu schalten, wenn ein Kriterium überprüft ist.

**12.** Vorrichtung nach vorangehendem Anspruch, wobei das mindestens eine Kriterium bei der Durchführung des Verfahrens nur Daten einbezieht, die sich auf die Unterstützungsvorrichtung beziehen.

**13.** Vorrichtung nach einem der Ansprüche 12 oder 13, wobei die Verarbeitungseinheit (U) ausgelegt ist, um zwei Impedanzen zu implementieren, die so parametriert sind, dass sie jeweils einen freien Modus und einen verriegelten Modus definieren, und ein Verriegelungskriterium implementiert, das den Wechsel aus dem freien Modus in den verriegelten Modus gestattet, und ein Entriegelungskriterium, das den Wechsel aus dem verriegelten Modus in den freien Modus gestattet.

**14.** Vorrichtung nach vorangehendem Anspruch, wobei das Verriegelungskriterium ein Stillstand des Instruments (20) während einer bestimmten Dauer ist und das Entriegelungskriterium eine Translation des Instruments (20) entlang der Instrumentenachse (X-X') ist.

## Claims

**1.** Device for assisting the manipulation of an instrument (20), comprising:
a hinged arm (10) intended to be fixed to a frame (B) and manipulable by an operator, to which an instrument (20) can be attached at an attachment point (P) of said hinged arm (10) forming a passive ball joint between the hinged arm (10) and the instrument (20), the passive ball joint transmitting no moment to the attachment point (P) of the instrument (20) and allowing free handling of the instrument (20) by the operator, the hinged arm comprising motors (M1, M2, ....) for displacing the attachment point (P) in a reference frame (R) bound to the frame (B), the instrument (20) being manipulable around a Fulcrum (F) having a known and fixed position in the reference frame (R),
a processing unit (U) comprising a processor (U1) configured to control the motors (M1, M2, ...); the device being configured to implement a preliminary step (E0) of determining a position of the fulcrum (F) in the reference frame (R) consisting in the steps of:

- Obtaining (E01), in the reference frame (R), a plurality of straight lines (Δ) defined by the instrument axis (X-X'), the straight lines corresponding to a plurality of positions of the said instrument (20) about the Fulcrum (F) and passing through the attachment point (P), each straight line being obtained after a given displacement of the attachment point (P);
- Estimating (E02) of an intersection zone ($V_{ol}$) of said plurality of straight lines (Δ),
- Obtaining (E03) the position of the center of said zone ($V_{ol}$), which corresponds to the fulcrum (F),

the device being configured to determine (E1) data relating to a position and/or velocity of the attachment point (P) in the reference frame (R);
the processing unit being configured for:

determining (E2, E3, E4) a force ($\overrightarrow{F_P}$) to be applied to the attachment point (P) so as to apply a given impedance at a point (Q) located on the instrument axis (X-X') connecting the attachment point (P) to the Fulcrum (F), the point (Q) being located at a known distance ($\overrightarrow{PQ}$) from the attachment point (P), the force to be applied being a function of said data relating to the attachment point (P), the position of the Fulcrum (F), the known distance ($\overrightarrow{PQ}$) from the attachment point (P) to the point (Q) , and of the given impedance to be applied to the point (Q) ;
the device also being configured to control (E5) motors (M1, M2, ...) to transmit the force ($\overrightarrow{F_P}$) determined at the instrument (20) at the attachment point (P), so as to produce the impedance at the point (Q).

**2.** Device according to the preceding claim, in which the processing unit (U) is configured to implement the step of determining the force ($\overrightarrow{F_P}$) to be applied to the attachment point in the following manner:

- determining (E2) velocity and/or position data of the point (Q) of the instrument located on an instrument axis (X-X') passing through the attachment point (P) and the Fulcrum (F), by means of said data relating to the attachment point (P) and the position of the Fulcrum (F);
- determining (E3) a force ($\overrightarrow{F_Q}$) to be applied to point (Q) as a function of an impedance to be imparted to the instrument (20) at said point (Q) of the instrument (20) and as a function of data determined from said point (Q) of the instrument (20);
- determining (E4) a force ($\overrightarrow{F_P}$) to be applied to the attachment point (P) as a function of the force ($\overrightarrow{F_Q}$) to be applied to the point (Q) and the data relating to the attachment point P and the position of the Fulcrum (F).

**3.** Device according to any one of the preceding claims, configured so that the impedance comprises a viscosity and the force ($\overrightarrow{F_P}$) applied to the attachment point (P) is a resistive force in the opposite direction to its displacement.

**4.** Device according to any one of the preceding claims, configured so that the impedance comprises a stiffness and the force ($\overrightarrow{F_P}$) applied to the attachment point (P) is a force in the same direction or the opposite direction to its displacement, depending on the sign of the stiffness.

5. Device according to the preceding claim, in which the articulated arm (10) comprises three articulations (A1, A2, A3) each actuated by a motor (M 1, M2, M3) and each comprising a sensor (C1, C2, C3) so as to determine the position of the attachment point (P) in the reference frame (R).

6. Device according to one of the preceding claims, in which the device (1) comprises two angular position sensors (C4, C5) at the attachment point (P) to obtain the orientation of the instrument axis (X-X').

7. A device according to any one of the preceding claims, wherein the processing unit (U) is configured so that the impedance includes a viscosity, and wherein the step (E3) of determining the force (Fq) at said point (Q) comprises the following steps for determining the impedance to be imparted:

- determining (E1') the instantaneous speed ($\vec{v}(Q)$) of said point (Q) of the instrument,
- determining (E21') a first viscosity as a decreasing function of the said instantaneous velocity ($\vec{v}(Q)$),
- determining (E22') a second viscosity from the first viscosity by means of a filtering process having at least one parameter enabling the dynamics of the process to be adjusted;
- determining (E3') the force at said point of the instrument, as a function of :

  ◦ of said velocity ($\vec{v}(Q)$), and
  ◦ of the second viscosity value used to adjust the dynamics.

8. A device according to the preceding claim, in which the processing unit (U) is configured so that the instantaneous speed of the point (Q) is filtered.

9. A device according to any one of the preceding claims, wherein the processing unit (U) is further configured to implement steps consisting of:

- having a point on the instrument coincide (E01') with points in space and determining the position of the said points in space in the frame of reference linked to the device for assisting the manipulation of an instrument;
- construct (E03') a geometric constraint defined by said points in space using said positions.

10. A device according to the preceding claim, wherein the processing unit (U) is configured so that the impedance comprises a stiffness, and wherein the processing unit is configured to implement a preliminary step of constructing a plane, said instrument point being a distal end, said step comprising thesub-steps of:

- Pointing (E01') with the distal end of at least three non-colinear points and determining their position, and determining (E02') their position in the reference frame (R),
- Construct (E03') a plane (PLAN1) passing through the three points using the three positions,
the step (E3) of determining the force ($\overrightarrow{F_P}$) at the attachment point (P) comprising the sub-steps of:

  - determining (E31") the distance between the said point (Q) on the instrument (20) and the plane by orthogonal projection,
  - determining (E32") the force ($\overrightarrow{F_Q}$) as a function of a stiffness and said distance.

so that the step (E5) of controlling the motors constrains the the instrument to position itself with respect to the plane (PLAN1) by causing the attraction or repulsion of said point (Q) of the instrument axis (X-X') with respect to said plane (PLAN1).

11. A device according to any one of the preceding claims, wherein a plurality of switching modes are implemented in the processing unit (U), each mode having a predetermined impedance, the processing unit (U) being configured to implement steps consisting of automatically switching from one control mode to another when a criterion is verified.

12. Device according to the preceding claim, in which the said at least one criterion involves only data relating to the said assistance device when the method is implemented.

13. Device according to any one of claims 12 or 13, in which the processing unit (U) is configured to implement two impedances which are parameterized for defining a free mode and a locked mode respectively, and implements a locking criterion which makes it possible to switch from the free mode to the locked mode and an unlocking criterion

which makes it possible to switch from the locked mode to the free mode.

**14.** Device according to the preceding claim in which the locking criterion is immobility of the instrument (20) for a given period of time and the unlocking criterion is translation of the instrument (20) along the instrument axis (X-X').

FIG. 1

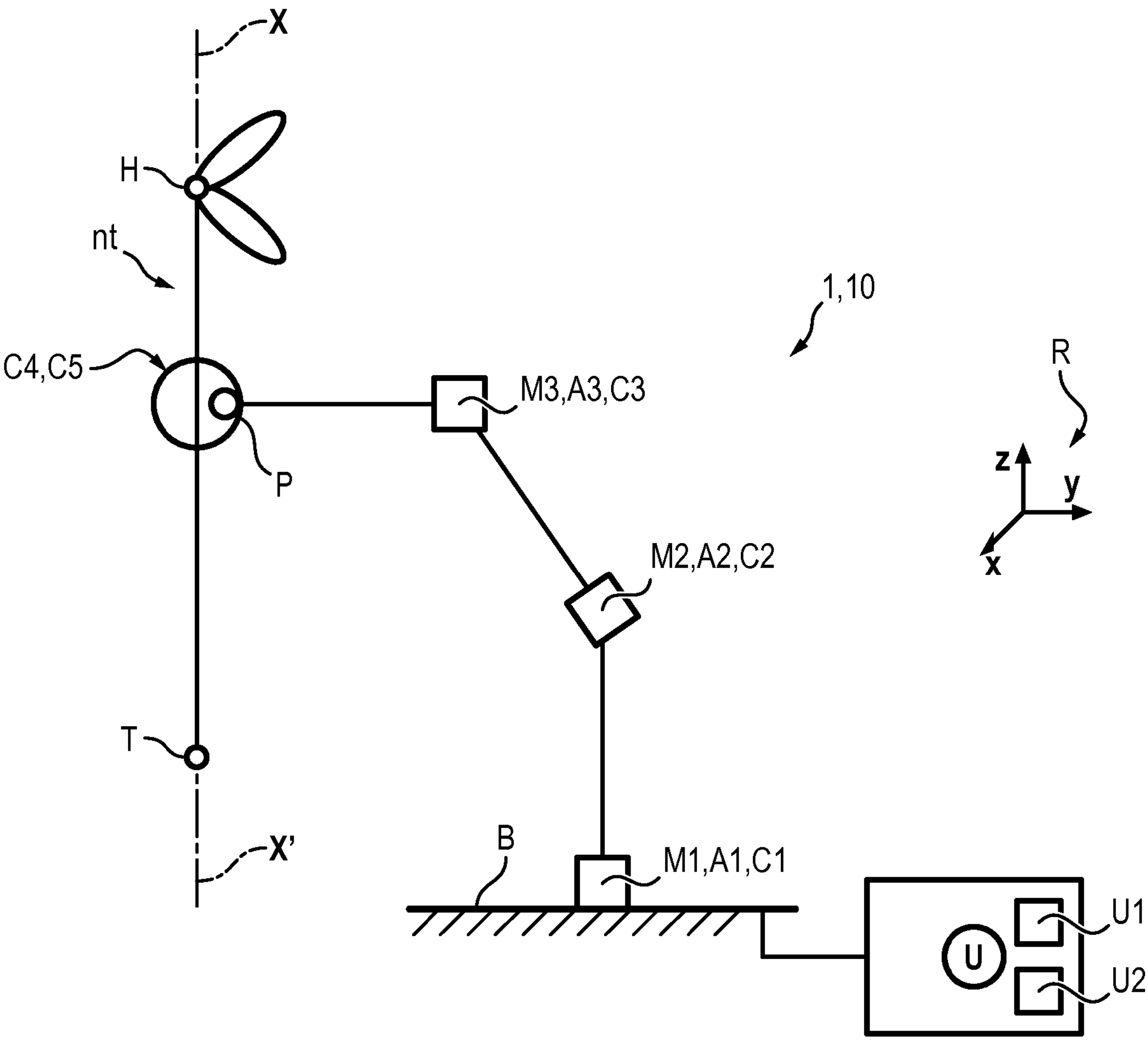

FIG. 2

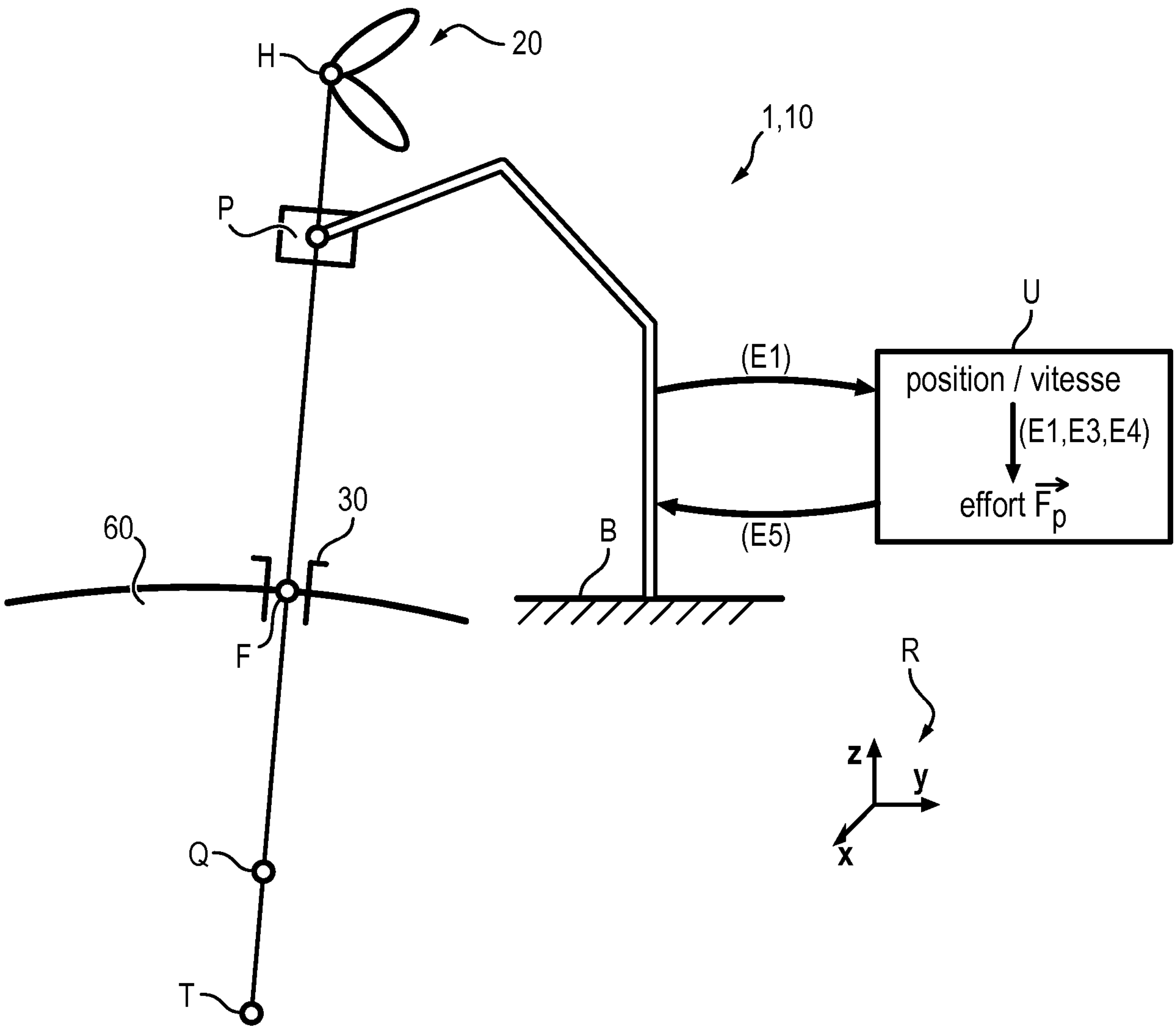
H
20
1,10
P
U
(E1)
position / vitesse
(E1,E3,E4)
effort $\vec{F_p}$
(E5)
B
60
30
F
R
z
y
x
Q
T

FIG. 3

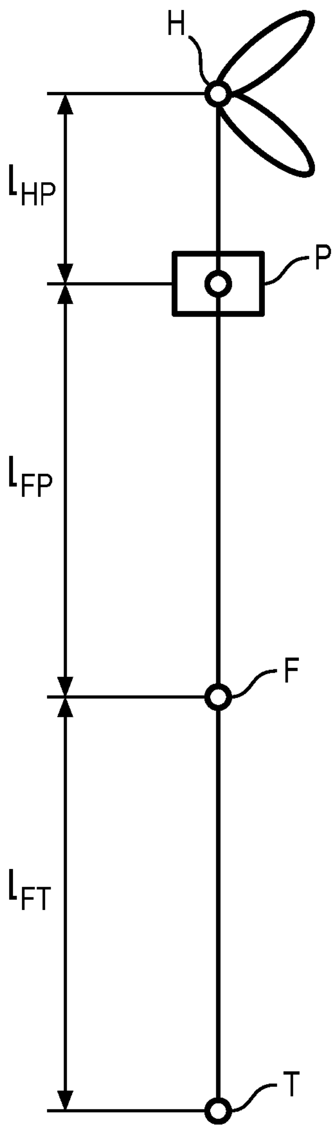

FIG. 4

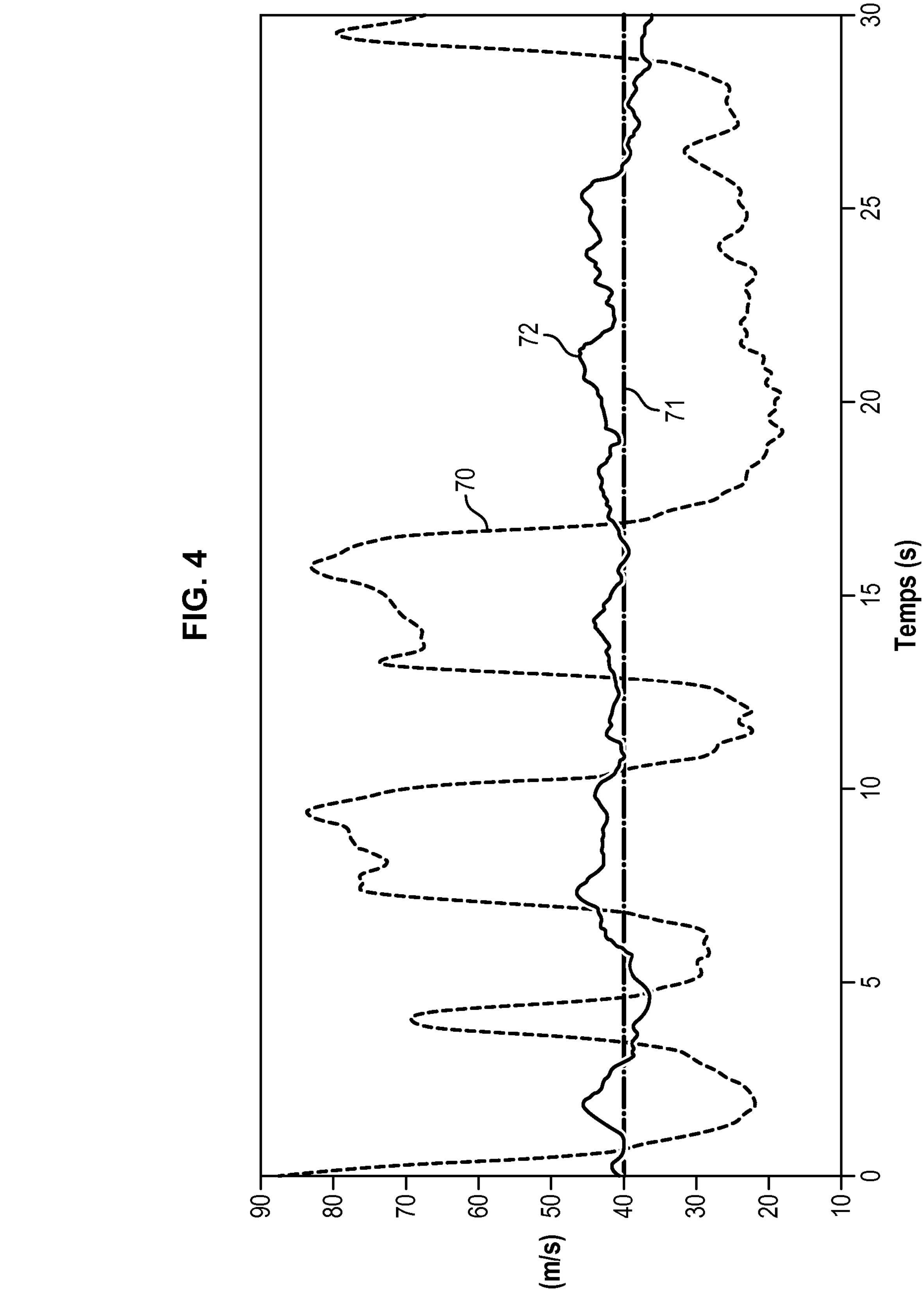
Vitesse (m/s)
90
80
70
60
50
40
30
20
10
Temps (s)
0
5
10
15
20
25
30
70
71
72

FIG. 5a

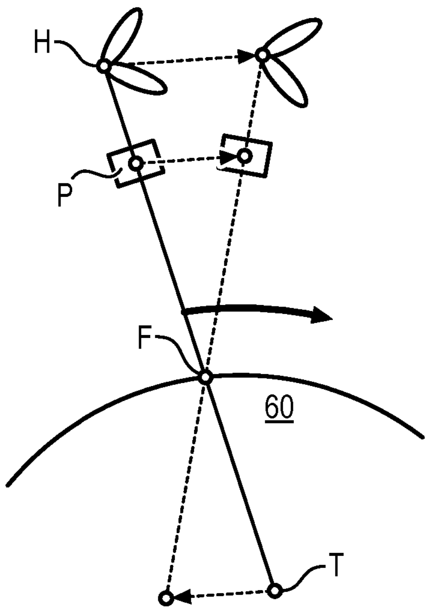


FIG. 5b

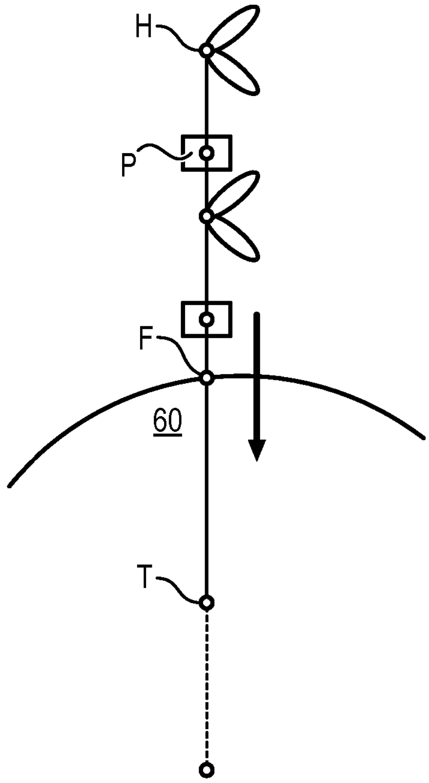


FIG. 6

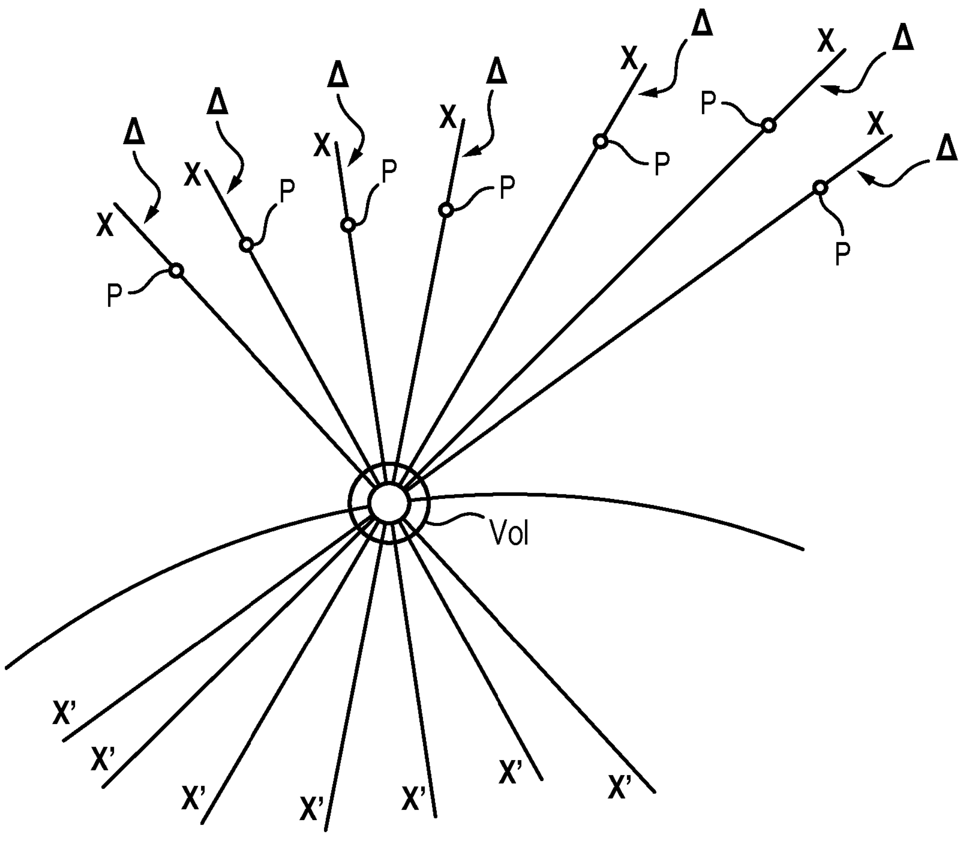

FIG. 7

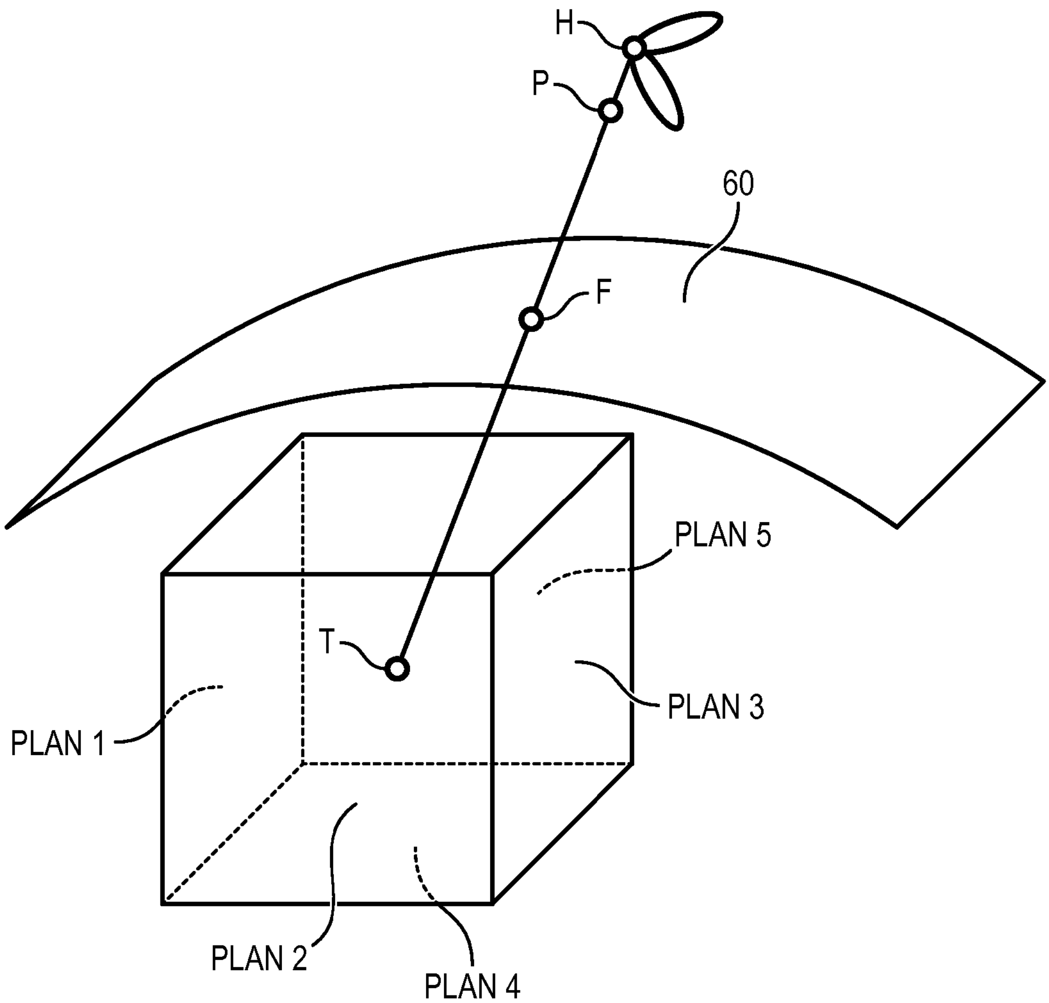

FIG. 8c

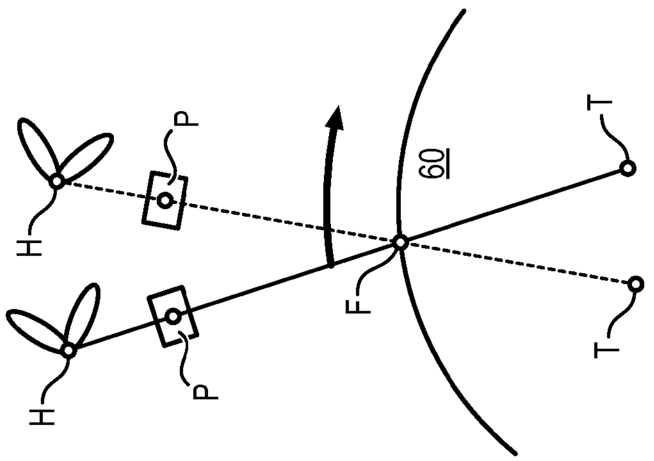


FIG. 8b

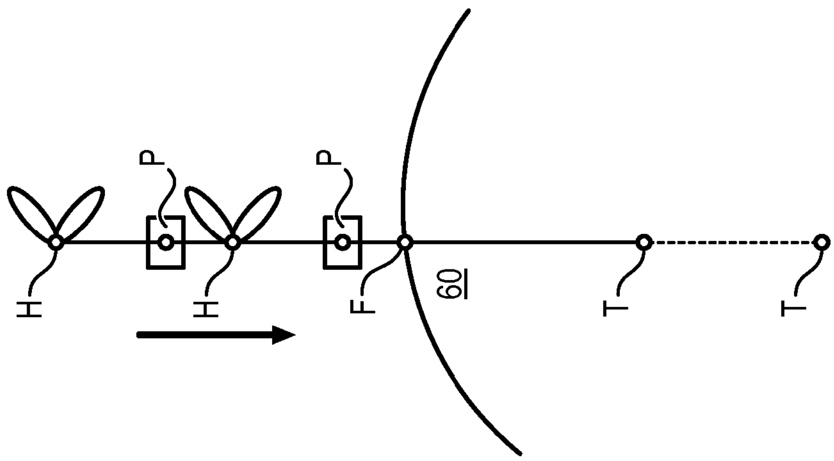


FIG. 8a

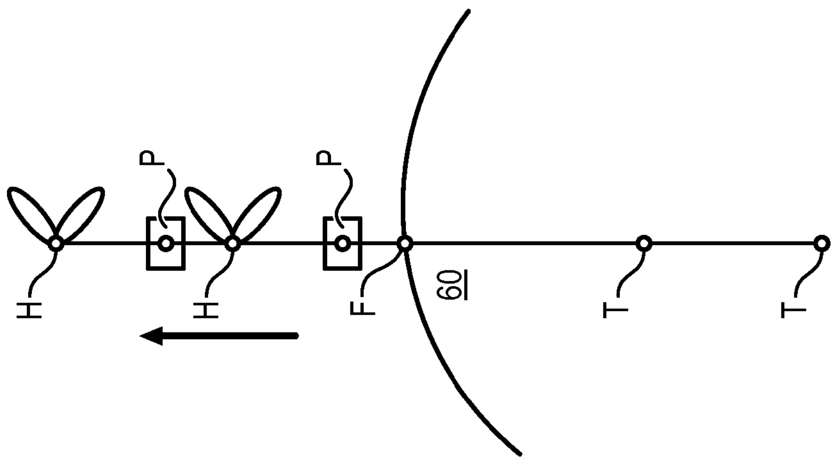

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006124390 A2
- US 6786896 B1
- US 2007142823 A1
- WO 02060653 A2